# EUROPEAN PATENT APPLICATION

(11) **EP 4 047 091 A1**
(43) Date of publication of application: **24.08.2022**
(21) Application number: 20875877.1
(22) Date of filing: 16.10.2020
(51) Int. Cl.: C12N 15/09, C12N 15/11, C12Q 1/6844, C12Q 1/6876, C12N 9/12, C12N 9/16

(54) **METHOD FOR DETECTING TARGET NUCLEIC ACID, METHOD FOR DETECTING NUCLEIC ACID-BINDING MOLECULE, AND METHOD FOR EVALUATING NUCLEIC ACID-BINDING ABILITY**

(30) Priority: 18.10.2019 JP 2019191409
(71) Applicant: Epigeneron, Inc., Tokyo, 103-0012 (JP)
(72) Inventor: FUJITA, Toshitsugu, Tokyo 103-0012 (JP); FUJII, Hodaka, Tokyo 103-0012 (JP)
(74) Representative: Troesch Scheidegger Werner AG
(86) International application number: PCT/JP2020/039128
(87) International publication number: WO 2021/075555

(57) **Abstract**

The present invention provides a method for detecting a target nucleic acid that discriminates the target nucleic acid from a non-target nucleic acid having a nucleotide sequence or modification state that differs from a portion of the target nucleic acid, the method comprising conducting a nucleic acid amplification reaction using a region in the non-target nucleic acid that differs from the target nucleic acid as a corresponding target region, using a region in the target nucleic acid that differs from the non-target nucleic acid as a target region, using a nucleic acid test sample as a template, and using a primer that hybridizes with both the target nucleic acid and the non-target nucleic acid, with the nucleic acid amplification reaction conducted in the presence of a molecule capable of binding specifically to the target region in the non-target nucleic acid, under temperature conditions under which the molecule can bind to the non-target nucleic acid, and then detecting the target nucleic acid on the basis of the presence or absence of an amplification product.

SEQUENCE LISTING

<110>  Epigeneron Inc.
<120>  PC-30396
<130>  Method for detecting target nucleic acid, method for detecting
molecule having nucleic acid binding ability, and method for evaluating
nucleic acid binding ability
<160>  38

<210>  1
<211>  42
<212>  RNA
<213>  Artificial Sequence
<220>
<223>  crRNA_KRAS
<400>  1
guaguuggag cugguggcgu guuuuagagc uaugcuguuu ug                    42

<210>  2
<211>  42
<212>  RNA
<213>  Artificial Sequence
<220>
<223>  crRNA_KRAS#2
<400>  2
cuugugguag uuggagcugg guuuuagagc uaugcuguuu ug                    42

<210>  3
<211>  42
<212>  RNA
<213>  Artificial Sequence
<220>
<223>  crRNA_hp16_Gx5#2
<400>  3
   acggccgcgg cccggggguc guuuuagagc uaugcuguuu ug                    42

<210>  4
<211>  42
<212>  RNA
<213>  Artificial Sequence
<220>
<223>  crRNA_KRAS_mut
<400>  4
guaguuggag cuggaggcgu guuuuagagc uaugcuguuu ug                    42

<210>  5
<211>  40
<212>  RNA
<213>  Artificial Sequence
<220>
<223>  crRNA_mid2
<400>  5
caccuccucu acccgacccc guuuuagagc uaugcuguuu                    40

<210>  6
<211>  69
<212>  RNA
<213>  Artificial Sequence
<220>
<223>  tracrRNA
<400>  6
aaacagcaua gcaaguuaaa auaaggcuag uccguuauca acuugaaaaa guggcaccga    60
gucggugcu                                                          69

<210>  7
<211>  34
<212>  DNA
<213>  Artificial Sequence
<220>
<223>  KRAS-RPA-G12-F primer
<400>  7
tagtgtatta accttatgtg tgacatgttc taat                                    34

<210>  8
<211>  34
<212>  DNA
<213>  Artificial Sequence
<220>
<223>  KRAS-RPA-G12-R primer
<400>  8
aaacaagatt tacctctatt gttggatcat attc                                    34


<210>  9
<211>  34
<212>  DNA
<213>  Artificial Sequence
<220>
<223>  p16-RPA-F2 primer
<400>  9
ggcggcgggg agcagcatgg agccttcggc tgac                                    34

<210>  10
<211>  34
<212>  DNA
<213>  Artificial Sequence
<220>
<223>  p16-RPA-R2 primer
<400>  10
ctacccacct ggatcggcct ccgaccgtaa ctat                                    34

<210>  11
<211>  30
<212>  DNA
<213>  Artificial Sequence
<220>
<223>  p14-RPA-F primer
<400>  11
gtcccagtct gcagttaagg gggcaggagt                                         30

<210>  12
<211>  28
<212>  DNA
<213>  Artificial Sequence
<220>
<223>  p14-RPA-R primer
<400>  12
gggcctttcc tacctggtct tctaggaa                                           28

<210>  13
<211>  32
<212>  DNA
<213>  Artificial Sequence
<220>
<223>  p16-RPA-F primer
<400>  13

gaggaagaaa gaggaggggc tggctggtca cc                                    32

<210>  14
<211>  32
<212>  DNA
<213>  Artificial Sequence
<220>
<223>  p16-RPA-R primer
<400>  14
ctgcagaccc tctacccacc tggatcggcc tc                                    32

<210>  15
<211>  23
<212>  DNA
<213>  Artificial Sequence
<220>
<223>  p14ARF-CpG_island-F primer
<400>  15
gtgggtccca gtctgcagtt aag                                              23

<210>  16
<211>  22
<212>  DNA
<213>  Artificial Sequence
<220>
<223>  p14ARF-CpG_island-R primer
<400>  16
acttttcgag ggcctttcct ac                                               22

<210>  17
<211>  30
<212>  DNA
<213>  Artificial Sequence
<220>
<223>  Pax5-LexA-RPA-F primer
<400>  17
gcatcagtcg cccttcgcct ccttctctcg                                       30

<210>  18
<211>  30
<212>  DNA
<213>  Artificial Sequence
<220>
<223>  Pax5-LexA-RPA-R primer
<400>  18
gcgagggcgg aacgtgactt tgccctgcgg                                       30

<210>  19
<211>  64
<212>  RNA
<213>  Artificial Sequence
<220>
<223>  gRNA_NEAT1
<400>  19
gauuuagacu accccaaaaa cgaaggggac uaaaaccauc aaucugcguu guggcaucaa      60
cguu                                                                   64

<210>  20
<211>  65
<212>  RNA
<213>  Artificial Sequence
<220>
<223>  gRNA_NEAT1_2

<400>  20
gauuuagacu accccaaaaa cgaaggggac uaaaacuauc ucuaaccaac ccucuccccu      60
ucuuc                                                                  65

<210>  21
<211>  20
<212>  DNA
<213>  Artificial Sequence
<220>
<223>  Human NEAT1-F2 primer
<400>  21
ctaaattgag cctccggtca                                                  20

<210>  22
<211>  20
<212>  DNA
<213>  Artificial Sequence
<220>
<223>  Human NEAT1-R2 primer
<400>  22
acaagaaggc aggcaaacag                                                  20

<210>  23
<211>  20
<212>  DNA
<213>  Artificial Sequence
<220>
<223>  MY-0119 primer
<400>  23
cactggtact gggagggatg                                                  20

<210>  24
<211>  22
<212>  DNA
<213>  Artificial Sequence
<220>
<223>  MY-0129 primer
<400>  24
cccttcaacc tgcatttcct ac                                               22

<210>  25
<211>  23
<212>  DNA
<213>  Artificial Sequence
<220>
<223>  hGAPDH-dCas13a-F3 primer
<400>  25
gagccaaaag ggtcatcatc tct                                              23

<210>  26
<211>  23
<212>  DNA
<213>  Artificial Sequence
<220>
<223>  hGAPDH-dCas13a-R3 primer
<400>  26
cacgatacca aagttgtcat gga                                              23

<210>  27
<211>  156
<212>  DNA
<213>  Artificial Sequence
<220>

<223>  Cis region in mouse Cis gene promoter
<400>  27
caactctagg agctcccgcc cagttttcct ggaaagttct tggaaatctg tcaaaggtgt      60
ttcctttctc ggtccaaagc actagacgcc tgcacccccg ttcccctccg ggccgccgca     120
aagcccgcgg ttctaggaag atgaggcttc cgggaa                               156

<210>  28
<211>  153
<212>  DNA
<213>  Artificial Sequence
<220>
<223>  CisM region
<400>  28
caactctagg agctcccgcc cagttggcct ggccagggct tggccatctg tcaaaggtgt      60
ttcctttctc ggtccaaagc actagacgcc tgcacccccg ttcccctccg ggccgccgca     120
aagcccgcgg ggctaggccg atgaggcggc caa                                  153

<210>  29
<211>  20
<212>  DNA
<213>  Artificial Sequence
<220>
<223>  mCis_-259/-199_F primer
<400>  29
caactctagg agctcccgcc                                                  20

<210>  30
<211>  19
<212>  DNA
<213>  Artificial Sequence
<220>
<223>  mCis_-188/-104_R primer
<400>  30
ttcccggaag cctcatctt                                                   19

<210>  31
<211>  52
<212>  DNA
<213>  Artificial Sequence
<220>
<223>  mCis_-259/-199_mut_F primer
<400>  31
caactctagg agctcccgcc cagttggcct ggccagggct tggccatctg tc              52

<210>  32
<211>  32
<212>  DNA
<213>  Artificial Sequence
<220>
<223>  mCis_-188/-104_mut_R primer
<400>  32
ggcccggccg cctcatcggc ctagccccgc gg                                    32

<210>  33
<211>  17
<212>  DNA
<213>  Artificial Sequence
<220>
<223>  M13 Primer RV primer
<400>  33
caggaaacag ctatgac                                                     17

<210>  34

<211>  17
<212>  DNA
<213>  Artificial Sequence
<220>
<223>  M13 Primer M4 primer
<400>  34
gttttcccag tcacgac                                                      17

<210>  35
<211>  33
<212>  DNA
<213>  Artificial Sequence
<220>
<223>  P5-NGS-Lib-Promega-RPA-F primer
<400>  35
atacggcgac caccgagatc tacactcttt ccc                                    33

<210>  36
<211>  33
<212>  DNA
<213>  Artificial Sequence
<220>
<223>  P7-NGS-Lib-Promega-RPA-R primer
<400>  36
caagcagaag acggcatacg agattctgac atg                                    33

<210>  37
<211>  22
<212>  DNA
<213>  Artificial Sequence
<220>
<223>  hp14ARF-Ex1-F primer
<400>  37
agtgagggtt ttcgtggttc ac                                                22

<210>  38
<211>  22
<212>  DNA
<213>  Artificial Sequence
<220>
<223>  hp14ARF-Ex1-R primer
<400>  38
cctagacgct ggctcctcag ta                                                22

## Description

### TECHNICAL FIELD

The present invention relates to a method for discriminating between a plurality of nucleic acids having similar nucleotide sequences or structures, such as in the cases of gene mutation, gene polymorphism or nucleic acid modification, and detecting a target nucleic acid, a method for detecting a nucleic acid-binding molecule and evaluating the nucleic acid-binding ability of that molecule, and a kit that may be used with these methods.

Priority is claimed on Japanese Patent Application No. 2019-191409, filed October 18, 2019, the content of which is incorporated herein by reference.

### BACKGROUND ART

The majority of gene mutations, in which the nucleotide sequence of a gene changes, involve the insertion, deletion, or conversion to another base, of between one and several bases. Accordingly, detection of gene mutations requires the discrimination and detection of a wild-type nucleic acid and a mutant nucleic acid which, with the exception of a mutation site of between one and several bases, is composed of the same nucleotide sequence.

A variety of techniques exist for detecting DNA or RNA mutations of between one and several bases. Examples of mutation detection methods that utilize PCR (polymerase chain reaction) include PCR methods using a modified oligonucleotide probe that rely on a fluorescent substance and a quencher. In such methods, the reaction for performing PCR amplification of a DNA fragment containing the DNA region predicted to include a mutation is conducted in the presence of an oligonucleotide probe, which is hybridized with the mutant DNA region predicted to include the mutation, and has been modified with a fluorescent substance and a quencher. The probe is annealed to the mutant DNA, the probe is cleaved by exonuclease activity of the DNA polymerase used in the PCR, and the resulting separation between the fluorescent substance and the quencher causes fluorescence in the reaction system. In the case of wild-type DNA, annealing of the probe does not occur, and therefore no fluorescence is generated. However, in the case of samples containing a mixture of wild-type and mutant DNA, complex operations are required to perform a quantitative evaluation, and therefore use of this method in the detection of heterozygous mutation is difficult.

Further, surveyor assay using surveyor nuclease is another possible method (see Non-Patent Document 1). In this assay, by mixing a PCR-amplified control DNA and test DNA in a test tube, conducting thermal denaturation and rehybridization, and then using surveyor nuclease to cleave the 3' side of the mismatched base, the inclusion of a base in the test DNA that differs from the control DNA can be detected. This method is comparatively simple, and is mainly used for verifying the efficiency of genome editing by extracting DNA from a cell population that has undergone gene editing. Typically, the genome editing efficiency is not 100% and there are various types of genome-edited sequences, meaning inclusion of a control DNA is unnecessary. However, in the detection of each genome edited cell, in order to detect a homozygous mutation, wild-type cell-derived DNA must be mixed. Furthermore, when analyzing each of the cell derivations, methods in which the nucleotide sequence is determined by the Sanger method or the like tend to be more direct, and therefore surveyor assay is not typically used.

Another method is the ORNi-PCR method, in which a short RNA (oligoribonucleotide, ORN) of approximately 17 to 29 bases that is complementary to the DNA region that is to be amplified by the PCR reaction is added to the reaction system (for example, see Non-Patent Document 2). In this method, PCR amplification of the DNA region hybridized with the ORN is specifically inhibited. In other words, only the DNA not hybridized with the ORN undergoes PCR amplification.

### PRIOR ART LITERATURE

### Patent Document

Patent Document 1: Japanese Unexamined Patent Application, First Publication No. 2011-103900

### Non-Patent Documents

Non-Patent Document 1: Zhu, et al, Scientific Reports, 2014, 4:6420, DOI: 10.1038/srep06420.
Non-Patent Document 2: Fujita, et al, DNA Research, 2018, vol.25, p.395-407.
Non-Patent Document 3: Notomi, et al, Nucleic Acids Research, 2000, vol.28(12), e63.
Non-Patent Document 4: Dean, et al, Proceedings of the National Academy of Sciences of the United States of America, 2002, vol.99(8), p.5261-5266.
Non-Patent Document 5: Fujita, et al. Scientific Reports, 2016, 6:30485.
Non-Patent Document 6: Fujita, et al. PLoS One, 2015, 10(6), e0116579.
Non-Patent Document 7: Abudayyeh, et al. Science, 2016, 353(6299), aaf5573.
Non-Patent Document 8: Gootenberg, et al. Science, 2017, 356(6336), p.438-442.
Non-Patent Document 9: Rascle and Lees, Nucleic Acids Research, 2003, vol.31(23), p.6882-6890.
Non-Patent Document 10: Abudayyeh, et al. Nature, 2017, vol.550(7675), p.280-284.

### SUMMARY OF INVENTION

### Problems to be Solved by the Invention

The main object of the present invention is to provide methods for detecting nucleic acid mutations and modifications, detecting molecules that have DNA-binding ability, and evaluating DNA-binding ability, by utilizing the inhibition of a nucleic acid amplification reaction by a molecule that binds to the template nucleic acid.

### Means for Solving the Problems

As a result of intensive research, the inventors of the present invention discovered that in a method such as RPA (Recombinase Polymerase Amplification) that enables amplification of a nucleic acid fragment having a target nucleotide sequence under isothermal conditions, by including, in the reaction system, a molecule that binds specifically to a nucleic acid having a specific nucleotide sequence or modification state, a nucleic acid that does not bind to the molecule can be specifically detected, thus enabling them to complete the present invention.

In other words, a method for detecting a target nucleic acid, a method for detecting a nucleic acid-binding molecule, a method for evaluating nucleic acid-binding ability, and a kit that may be used in these methods according to the present invention include the following aspects.
[1] A method for detecting a target nucleic acid that discriminates the target nucleic acid from a non-target nucleic acid having a nucleotide sequence or modification state that differs from a portion of the target nucleic acid, the method comprising conducting a nucleic acid amplification reaction:
   using a region in the non-target nucleic acid in which the nucleotide sequence or modification state differs from that of the target nucleic acid as a target region,
   using a region in the target nucleic acid in which the nucleotide sequence or modification state differs from that of the non-target nucleic acid as a corresponding target region,
   using a nucleic acid test sample as a template, and
   using a primer that hybridizes with both the target nucleic acid and the non-target nucleic acid,
   with the nucleic acid amplification reaction conducted in the presence of a molecule (excluding molecules composed solely of a nucleic acid) capable of binding specifically to the target region in the non-target nucleic acid, under temperature conditions under which the molecule can bind to the non-target nucleic acid, and then
   detecting the target nucleic acid on the basis of the presence or absence of an amplification product.
[2] The method for detecting a target nucleic acid according to [1] above, wherein when an amplification product is obtained in the nucleic acid amplification reaction, the target nucleic acid is contained in the nucleic acid test sample.
[3] The method for detecting a target nucleic acid according to [1] or [2] above, wherein the nucleic acid amplification reaction is conducted under temperature conditions of 65°C or lower.
[4] The method for detecting a target nucleic acid according to any one of [1] to [3] above, wherein the nucleic acid amplification reaction is an isothermal nucleic acid amplification reaction.
[5] The method for detecting a target nucleic acid according to any one of [1] to [4] above, wherein the nucleic acid amplification reaction uses the Recombinase Polymerase Amplification method.
[6] The method for detecting a target nucleic acid according to any one of [1] to [5] above, wherein the corresponding target region in the target nucleic acid and the target region in the non-target nucleic acid have different nucleotide sequences.
[7] The method for detecting a target nucleic acid according to [6] above, wherein the target region is a mutation site of a gene mutation or a polymorphic site of a gene polymorphism.
[8] The method for detecting a target nucleic acid according to any one of [1] to [7] above, wherein the molecule is a complex of a DNA strand cleavage activity-deficient Cas9 protein and a gRNA.
[9] The method for detecting a target nucleic acid according to [8] above, wherein the gRNA specifically recognizes and binds to DNA having a nucleotide sequence complementary to the target region in the non-target nucleic acid.
[10] The method for detecting a target nucleic acid according to any one of [1] to [9] above, wherein
   the nucleic acid test sample has been subjected to a bisulfite treatment, and
   the methylation states of the corresponding target region and the target region differ, and include bases that yield a difference between the corresponding target region and the target region as a result of the bisulfite treatment.
[11] The method for detecting a target nucleic acid according to any one of [1] to [7] above, wherein the molecule is a complex of an RNA strand cleavage activity-deficient Cas13a protein and a gRNA.
[12] The method for detecting a target nucleic acid according to [11] above, wherein the gRNA specifically recognizes and binds to RNA having a nucleotide sequence complementary to the target region in the non-target nucleic acid.
[13] The method for detecting a target nucleic acid according to any one of [1] to [5] above, wherein the corresponding target region in the target nucleic acid and the target region in the non-target nucleic acid have different modification states.
[14] The method for detecting a target nucleic acid according to [13] above, wherein
   the corresponding target region in the target nucleic acid is a region that has not undergone CpG methylation modification,
   the target region in the non-target nucleic acid is a region that has undergone CpG methylation modification, and
   the molecule is a CpG methylated DNA-binding protein.
[15] A target nucleic acid detection kit used in the method for detecting a target nucleic acid according to any one of [8] to [10] above, the kit having
   a primer that hybridizes with both the target nucleic acid and the non-target nucleic acid,
   a DNA strand cleavage activity-deficient Cas9 protein, and
   a gRNA.
[16] A target nucleic acid detection kit used in the method for detecting a target nucleic acid according to [14] above, the kit having
   a primer that hybridizes with both the target nucleic acid and the non-target nucleic acid, and
   a CpG methylated DNA-binding protein.
[17] The target nucleic acid detection kit according to [15] or [16] above, further including a recombinase, a single-stranded DNA-binding protein, and a DNA polymerase.
[18] A target nucleic acid detection kit used in the method for detecting a target nucleic acid according to [11] or [12] above, the kit having
   a primer that hybridizes with both the target nucleic acid and the non-target nucleic acid,
   an RNA strand cleavage activity-deficient Cas13a protein, and
   a gRNA.
[19] A method for detecting a nucleic acid-binding molecule, the method comprising:
   conducting a nucleic acid amplification reaction using a test sample, a nucleic acid, and a primer that hybridizes with the nucleic acid, wherein
   when the nucleic acid-binding molecule is contained within the test sample, an amplification reaction product is not obtained from the nucleic acid amplification reaction, whereas when the nucleic acid-binding molecule is not contained within the test sample, an amplification reaction product is obtained from the nucleic acid amplification reaction.
[20] The method for detecting a nucleic acid-binding molecule according to [19] above, wherein the nucleic acid-binding molecule is a molecule that binds to a specific nucleic acid sequence.
[21] The method for detecting a nucleic acid-binding molecule according to [19] above, wherein the nucleic acid-binding molecule is a CpG methylated DNA-binding protein.
[22] A nucleic acid-binding molecule detection kit used in the method for detecting a nucleic acid-binding molecule according to any one of [19] to [21], the kit having
   a nucleic acid, and
   a primer that hybridizes with the nucleic acid.
[23] A method for evaluating nucleic acid-binding ability to a nucleic acid of a test substance, the method comprising
   conducting a nucleic acid amplification reaction, using a test substance, a target nucleic acid that evaluates the nucleic acid-binding ability of the test substance, and a primer that hybridizes with the nucleic acid, under temperature conditions under which the test substance can bind to the nucleic acid, wherein
   when an amplification product is obtained, the test substance is evaluated as not having binding ability to the nucleic acid, whereas when an amplification product is not obtained, the test substance is evaluated as having binding ability to the nucleic acid.
[24] The method for evaluating nucleic acid-binding ability according to [23] above, wherein the test substance is a complex of a DNA strand cleavage activity-deficient Cas9 protein and a gRNA.
[25] The method for evaluating nucleic acid-binding ability according to [23] above, wherein the nucleic acid is a CpG methylated DNA.
[26] A nucleic acid-binding ability evaluation kit used in the method for evaluating nucleic acid-binding ability according to any one of [23] to [25] above, the kit having
   a target nucleic acid that evaluates the nucleic acid-binding ability of the test substance, and
   a primer that hybridizes with the nucleic acid.

### Effects of the Invention

By employing the method for detecting a target nucleic acid according to the present invention, a thermal cycler that is typically required for conducting temperature control in PCR is unnecessary, and the target nucleic acid can be detected as a positive signal for the nucleic acid amplification product.

Further, by using the target nucleic acid detection kit according to the present invention, a simpler target nucleic acid detection method can be used to detect the target nucleic acid.

By using the method for detecting a nucleic acid-binding molecule and the method for evaluating nucleic acid-binding ability according to the present invention, nucleic acid-binding ability can be detected and evaluated using the degree of reduction in the nucleic acid amplification product as an indicator.

Furthermore, by using the nucleic acid-binding molecule detection kit and the nucleic acid-binding ability evaluation kit according to the present invention, a simpler method for detecting the nucleic acid-binding molecule and a simpler method for evaluating nucleic acid-binding ability can be realized.

### BRIEF DESCRIPTION OF THE DRAWINGS

FIG. 1 is a diagram schematically illustrating one aspect, among the principles of the method for detecting a target nucleic acid according to the present invention, for detecting a gene mutation using a DNA strand cleavage activity-deficient Cas9 protein (dCas9) and a gRNA as the target region-specific binding molecule.
FIG. 2 is a diagram schematically illustrating a method, among the principles of the method for detecting a target nucleic acid according to the present invention, for detecting a gene mutation by RT-PCR with an RNA as the target nucleic acid. A complex of an RNA strand cleavage activity-deficient Cas13a protein (dCas13a) and a gRNA was used as the target region-specific binding molecule.
FIG. 3 is a diagram illustrating the nucleotide sequences of a partial region of the *KRAS* gene of a 293T cell and two types of gRNA used in Example 1.
FIG. 4 is a staining image of the separated bands in Example 1 when an RPA reaction was conducted together with the each type of gRNA and dCas9, and the obtained reaction mixture was subjected to agarose electrophoresis.
FIG. 5 is a staining image of the separated bands in Example 2 when an RPA reaction was conducted together with the each type of gRNA and dCas9, and the obtained reaction mixture was subjected to agarose electrophoresis.
FIG. 6 is a diagram schematically illustrating the RPA reaction conducted in the reaction mixture to which gRNA_KRAS#2 had been added in Example 2.
FIG. 7 is a diagram illustrating the nucleotide sequence of gRNA_KRAS_mut having a single base substitution mutation introduced into gRNA-KRAS in Example 2.
FIG. 8 is a staining image of the separated bands in Example 2 when an RPA reaction was conducted together with gRNA_KRAS#2 and dCas9, and the obtained reaction mixture was subjected to agarose electrophoresis.
FIG. 9 is a diagram illustrating the nucleotide sequences of a partial region of the *CDKN2A (p16)* gene of an HCT116 cell and the gRNA_p16_Gx5#2 used in Example 3.
FIG. 10 is a staining image of the separated bands in Example 3 when an RPA reaction was conducted together with gRNA_KRAS or gRNA_p16_Gx5#2 and dCas9, and the obtained reaction mixture was subjected to agarose electrophoresis.
FIG. 11 is a diagram schematically illustrating genome editing of the *CDKN2A (p16)* gene in Example 4, and also schematically illustrating the RPA reaction conducted in the reaction mixture to which gRNA_mid2 had been added.
FIG. 12 is a staining image of the separated bands in Example 4 when an RPA reaction was conducted together with gRNA_mid2 and dCas9, and the obtained reaction mixture was subjected to agarose electrophoresis.
FIG. 13 is a staining image of the separated bands in Example 5 when an RPA reaction was conducted together with gRNA_KRAS#2 and dCas9, and the obtained reaction mixture was subjected to agarose electrophoresis.
FIG. 14 is a staining image of the separated bands in Example 5 when an RPA reaction was conducted together with gRNA_KRAS#2 and dCas9, and the obtained reaction mixture was subjected to agarose electrophoresis.
FIG. 15 is a staining image of the separated bands in Example 6 when an RPA reaction was conducted together with the MBD2 protein, and the obtained reaction mixture was subjected to agarose electrophoresis.
FIG. 16 is a staining image of the separated bands in Example 6 when an RPA reaction was conducted together with the MBD2 protein, and the obtained reaction mixture was subjected to agarose electrophoresis.
FIG. 17 is a staining image of the separated bands in Example 6 when an RPA reaction was conducted together with the MBD2 protein, and the obtained reaction mixture was subjected to agarose electrophoresis.
FIG. 18 is a staining image of the separated bands in Example 7 when an RPA reaction was conducted together with the LexA protein or dCas9, and the obtained reaction mixture was subjected to agarose electrophoresis.
FIG. 19 is a staining image of the separated bands in Example 8 when mRNA (NEAT1-RNA) of the human *NEAT1* gene was used as the target nucleic acid, RT-PCR was conducted together with gRNA_NEAT1 and dCas13a, and the obtained reaction mixture was subjected to agarose electrophoresis.
FIG. 20 is a staining image of the separated bands in Example 8 when mRNA (NEAT1-RNA) of the human *NEAT1* gene was used as the target nucleic acid, RT-PCR was conducted together with gRNA_NEAT1_2 and dCas13a, and the obtained reaction mixture was subjected to agarose electrophoresis.
FIG. 21 is a staining image of the separated bands in Example 9 when a Cis plasmid or CisM plasmid was used as a template, an RPA reaction was conducted in the presence of a nuclear extract that had either undergone no IL-3 treatment or had been subjected to IL-3 treatment, and the obtained reaction mixture was subjected to agarose electrophoresis.

### EMBODIMENTS FOR CARRYING OUT THE INVENTION

### <Method for Detecting Target Nucleic Acid>

In the present invention and the present description, the term "target nucleic acid" describes a nucleic acid that is the target of detection. There are no particular limitations on the target nucleic acid, provided the nucleic acid has a nucleotide sequence that is specified sufficiently to enable design of a primer that can be used in a nucleic acid amplification reaction using the primer and a polymerase. Specifically, single-stranded DNA, double-stranded DNA, single-stranded RNA, and RNA-DNA hybrids (nucleic acids in which a single-stranded RNA and a single-stranded DNA form a double strand) may also be used as the target nucleic acid. Further, these target nucleic acids include nucleic acids having a specific modification state. Examples of this modification state include methylation, fluorination, phosphorothioation, phosphorodithioation, sugar addition, PEG (polyethylene glycol) addition, and peptide addition.

In the present invention and the present description, the term "non-target nucleic acid" means a nucleic acid having a similar structure to the target nucleic acid, but being partially different from the target nucleic acid in terms of the nucleotide sequence or the modification state. There are no particular limitations on the non-target nucleic acid, provided the nucleotide sequence or modification state that represents the structural difference from the target nucleic acid is well-defined, enabling the nucleic acid to be readily discriminated from the target nucleic acid. For example, a nucleic acid having a CpG methylation modification may be used as the target nucleic acid, with a nucleic acid composed of a similar nucleotide sequence to the target nucleic acid but having no CpG methylation modification then used as the non-target nucleic acid.

In the present invention and the present description, the term "corresponding target region" means a region in the target nucleic acid having a different structure from that in the non-target nucleic acid, whereas the term "target region" means a region in the non-target nucleic acid having a different structure from that in the target nucleic acid. The position in which the corresponding target region is located within the target nucleic acid corresponds with the position in which the target region is located within the non-target nucleic acid. For example, in those cases where the structural difference between the target nucleic acid and the non-target nucleic acid is a difference in nucleotide sequence, the site (difference site) in the target nucleic acid having a different nucleotide sequence from that of the non-target nucleic acid is the corresponding target region, and the site (difference site) in the non-target nucleic acid having a different nucleotide sequence from that of the target nucleic acid is the target region. Similarly, in those cases where the structural difference between the target nucleic acid and the non-target nucleic acid is a difference in modification state, the site (difference site) in the target nucleic acid having a different modification state from that of the non-target nucleic acid is the corresponding target region, and the site (difference site) in the non-target nucleic acid having a different modification state from that of the target nucleic acid is the target region. The corresponding target region in the target nucleic acid may be a single region or two or more regions.

In the present invention and the present description, the term "target nucleotide sequence" means the nucleotide sequence of a region containing the corresponding target region in the target nucleic acid. The target nucleotide sequence may be a nucleotide sequence composed solely of the corresponding target region, may be the nucleotide sequence of a partial region of the target nucleic acid that includes the corresponding target region, or may be the nucleotide sequence of the entire length of the target nucleic acid.

In the present invention and the present description, the expression that "nucleotide sequences are homologous" means that "the nucleotide sequences are the same", whereas the expression that "nucleotide sequences are complementary" means that "the nucleotide sequences are mutually complementary".

In the detection of a nucleic acid containing a specific nucleotide sequence, the detection sensitivity can be enhanced by amplifying the region containing the nucleotide sequence via a nucleic acid amplification reaction, and detecting the amplification product. In a detection method that utilizes this type of nucleic acid amplification reaction, sometimes amplification occurs of not only the nucleic acid fragment composed of the nucleotide sequence of the detection target, but also nucleic acid fragments having nucleotide sequences similar to the target nucleotide sequence. In a nucleic acid amplification reaction, the detection precision of the nucleic acid fragment composed of the nucleotide sequence of the detection target can be enhanced by inhibiting amplification of nucleic acid fragments having nucleotide sequences similar to the nucleotide sequence of the detection target.

In the method for detecting a target nucleic acid according to the present invention, a nucleic acid amplification reaction is conducted using a primer and a polymerase with a nucleic acid test sample as the template, and the target nucleic acid in the nucleic acid test sample is detected as a nucleic acid amplification product. In the method for detecting a target nucleic acid according to the present invention, this nucleic acid amplification reaction is conducted in the presence of a molecule (excluding molecules composed solely of a nucleic acid) that binds to a target region in a non-target nucleic acid but does not bind to a corresponding target region in the target nucleic acid. Hereafter, this "molecule (excluding molecules composed solely of a nucleic acid) that binds to a target region in a non-target nucleic acid but does not bind to a corresponding target region in the target nucleic acid" is sometimes referred to as the "target region-specific binding molecule". In a nucleic acid amplification reaction that uses the non-target nucleic acid to which this target region-specific binding molecule has been bound as a template, the presence of the target region-specific binding molecule that is bound to the target region inhibits annealing of the primer to the template nucleic acid and the nucleic acid extension reaction promoted by the polymerase are inhibited. As a result, nucleic acid amplification of the non-target nucleic acid is inhibited, the proportion of amplification product of the target nucleic acid within the overall amplification product is increased, and the detection precision for the target nucleic acid improves.

Specifically, the method for detecting a target nucleic acid according to the present invention is a method that discriminates and detects a target nucleic acid from a non-target nucleic acid, wherein the method comprises conducting a nucleic acid amplification reaction, in the presence of a target region-specific binding molecule, using a nucleic acid test sample as a template and using a primer that hybridizes with both the target nucleic acid and the non-target nucleic acid, and then detecting the target nucleic acid on the basis of the presence or absence of an amplification product. As a result of the nucleic acid amplification reaction, the target nucleic acid is amplified, and therefore when the nucleic acid test sample contains the target nucleic acid, an amplification product is obtained from the nucleic acid amplification reaction. In other words, the target nucleic acid within the nucleic acid test sample is detected as an amplification product of the nucleic acid amplification reaction.

In the present invention, there are no particular limitations on the target nucleic acid and the non-target nucleic acid, provided they have regions of mutually different structures located within a common structure. The method for detecting a target nucleic acid according to the present invention is able to detect even a small difference in the nucleic acid structure, and therefore in those cases where the structural difference between the target nucleic acid and the non-target nucleic acid is a difference in the nucleotide sequence, the corresponding target region within the target nucleic acid is preferably a region of not more than 20 bases, more preferably a region of not more than 10 bases, even more preferably a region of 1 to 5 bases, and still more preferably a region of either 1 or 2 bases.

In the present invention and the present description, the term "nucleic acid test sample" is a sample containing nucleic acid that is tested for detection of the target nucleic acid. In the present invention, the nucleic acid within the nucleic acid test sample is analyzed to detect whether or not the target nucleic acid is included. There are no particular limitations on the nucleic acid test sample, provided it is a sample that contains nucleic acid. For example, nucleic acids extracted from animals, plants, microbes, viruses, or cultured cells or the like may be used as the nucleic acid test sample. Further, chemically synthesized nucleic acids and nucleic acids that have been subjected to intentional modification treatments may also be used as the nucleic acid test sample. Extraction of nucleic acids from cells or the like can be conducted using conventional techniques such as the phenol/chloroform method.

There are no particular limitations on the nucleic acid contained in the nucleic acid test sample, provided it can function as a template for the performed nucleic acid amplification reaction. Widely used nucleic acid amplification reactions that use DNA as the template can be utilized, and therefore the nucleic acid in the nucleic acid test sample supplied to the method for detecting a target nucleic acid according to the present invention is preferably DNA, and more preferably double-stranded DNA. The nucleic acid in the nucleic acid test sample supplied to the method for detecting a target nucleic acid according to the present invention may also be RNA. In those cases where the nucleic acid in the nucleic acid test sample is RNA, a reverse transcription reaction using the RNA as a template may be employed as the nucleic acid amplification reaction in the method for detecting a target nucleic acid according to the present invention, or alternatively, cDNA may first be synthesized by a reverse transcription reaction, and subsequently supplied to the nucleic acid amplification reaction.

In the present invention, the primer used for amplifying the target nucleic acid in the nucleic acid amplification reaction hybridizes with both the target nucleic acid and the non-target nucleic acid. Accordingly, in the absence of the target region-specific binding molecule, the nucleic acid amplification reaction using the primer also amplifies the non-target nucleic acid contained in the nucleic acid test sample. In the present invention, by conducting the nucleic acid amplification reaction in the presence of the target region-specific binding molecule, nucleic acid amplification of the non-target nucleic acid is inhibited. In the present invention, the primer used for amplifying the target nucleic acid in the nucleic acid amplification reaction may be any primer that hybridizes with both the target nucleic acid and the non-target nucleic acid under the conditions under which the nucleic acid amplification reaction is conducted, and may be a primer that hybridizes with a partial region within the target nucleic acid having a nucleotide sequence or modification state that is the same as that of the non-target nucleic acid, or may be a primer that hybridizes with a partial region within the target nucleic acid having a nucleotide sequence or modification state that differs from that of the non-target nucleic acid.

In the present invention, the primer for amplifying the target nucleic acid used in the nucleic acid amplification reaction is an oligonucleotide in which nucleotides are bonded via phosphate diester bonds, or a modified product of such an oligonucleotide. These primers may be composed solely of natural nucleotides (naturally occurring nucleotides) such as DNA and RNA, may be composed solely of artificial nucleotides prepared by modifying a natural nucleotide to form a nucleotide capable of phosphate diester bonding with a natural nucleotide, or may be a chimera molecule containing both a natural nucleotide and an artificial nucleotide. Examples of artificial nucleotides include nucleotides in which a side chain or the like of a natural nucleotide has been modified with a functional group such as an amino group, nucleotides in which the hydroxy group at the 2' position of a ribose skeleton has been substituted with a methoxy group, fluoro group, or methoxyethyl group or the like, phosphorothioate nucleotides (nucleotides in which the oxygen atom of a phosphate group has been substituted with a sulfur atom), morpholino nucleotides (nucleotides in which a ribose or deoxyribose has been substituted with a morpholine ring), BNA (Bridged Nucleic Acid), HNA (Hexitol Nucleic Acid), LNA (Locked Nucleic Acid), PNA (Peptide Nucleic Acid), TNA (Threose Nucleic Acid), GNA (Glycerol Nucleic Acid), and CeNA (Cyclohexenyl Nucleic Acid) and the like. Further, examples of modified products of these oligonucleotides include oligonucleotides that have been modified with a labeling substance to assist the detection of the amplification product. Examples of these labeling substances include fluorescent substances, radioisotopes, chemiluminescent materials, enzymes, and antibodies and the like. The labeling substance should be bonded so as not to impede the nucleic acid extension reaction of the primer.

In the present invention, the primer used in the nucleic acid amplification reaction may be a single primer, or may include two primers, or three or more primers. The primer(s) may be selected appropriately in accordance with the nucleic acid amplification reaction to be conducted.

For example, a primer set composed of a forward primer and a reverse primer may be used. For example, the target nucleotide sequence may be the nucleotide sequence of the entire length of, or a partial region of, the target nucleic acid, and regions having the same structure as the non-target nucleic acid, specifically regions having the same nucleotide sequence and modification state as the non-target nucleic acid, may be set at both the 5' side and the 3' side of the corresponding target region. In this case, a primer set may be used for amplifying the nucleic acid fragment of the target nucleotide sequence, composed of a forward primer that hybridizes with the region that represents the 5' end of the target nucleotide sequence and has the same structure as the non-target nucleic acid, and a primer that hybridizes with the region that represents the 3' end of the target nucleotide sequence and has the same structure as the non-target nucleic acid.

In the method for detecting a target nucleic acid according to the present invention, the nucleic acid amplification reaction is conducted under temperature conditions under which the target region-specific binding molecule can bind to the non-target nucleic acid. The nucleic acid amplification reaction may be conducted under any temperature conditions under which the target region-specific binding molecule can bind to the non-target nucleic acid throughout the entire reaction process, and may include temperature cycling such as in the PCR method.

In the method for detecting a target nucleic acid according to the present invention, the nucleic acid amplification reaction is preferably conducted under temperature conditions of 65°C or lower. By conducting the reaction under temperature conditions of 65°C or lower, molecules having comparatively low heat resistance such as proteins or the like can be used as the target region-specific binding molecule. By using a target region-specific binding molecule composed of a heat-resistant protein, and using a heat-resistant polymerase or the like, the nucleic acid amplification reaction can also be conducted at a temperature exceeding 65°C, for example, in a temperature range exceeding 65°C but not more than about 95°C.

Because a thermal cycler that is required for conducting temperature control in PCR is unnecessary, the nucleic acid amplification reaction in the method for detecting a target nucleic acid according to the present invention is preferably conducted under isothermal conditions. Here, the term "isothermal conditions" means that the temperature during the reaction is maintained within a range of ±3°C or ±1°C from the set temperature.

Although there are no particular limitations on the isothermal conditions, provided the nucleic acid amplification reaction proceeds satisfactorily, the constant temperature is, for example, a fixed temperature within the optimal temperature range for the DNA polymerase. Examples of the isothermal conditions include a fixed temperature that is at least 10°C, at least 15°C, at least 20°C, at least 25°C, or at least 30°C, and not more than 65°C, not more than 60°C, not more than 50°C, not more than 45°C, or 40°C or lower. Further, the isothermal conditions may be a fixed temperature included within a range from 10°C to 65°C, a fixed temperature included within a range from 15°C to 50°C, a fixed temperature included within a range from 20°C to 45°C, a fixed temperature included within a range from 20°C to 45°C, or a fixed temperature included within a range from 30°C to 45°C. In this description, expressions such as "incubate at a constant temperature", "hold the temperature under isothermal conditions", and "react at a constant temperature" mean that the temperature is held within a temperature range of ±7°C, ±5°C, ±3°C or ±1°C relative to the temperature that has been set for the reaction.

In the method for detecting a target nucleic acid according to the present invention, there are no particular limitations on the nucleic acid amplification reaction conducted under isothermal conditions, and conventional isothermal nucleic acid amplification reactions or modified reactions thereof may be used. Examples of conventional isothermal nucleic acid amplification reactions include the RPA method (Patent Document 1), the LAMP (Loop-Mediated Isothermal Amplification method (Non-Patent Document 3), the MDA (Multiple Displacement Amplification) method (Non-Patent Document 4), reverse transcription reactions using a reverse transcription enzyme, and the NASBA (Nucleic Acid Sequence-Based Amplification) method.

The RPA method is a method in which a primer that hybridizes with one end region of the target nucleic acid and a primer that hybridizes with the other end region of the target nucleic acid each form a complex with a recombinase which is brought into contact with the template nucleic acid, and following formation of a replication fork together with a single-stranded DNA-binding protein (SSB), a double-stranded nucleic acid is synthesized by a DNA polymerase. The primers used can be designed in the same manner as PCR primers. The SSB, DNA polymerase, and the buffer and the like used in preparing the reaction mixture may be selected appropriately from among substances typically used in the RPA method or modified products of these substances. Further, commercially available RPA kits such as TwistAmp (a registered trademark) (manufactured by TwistDx Ltd.) may also be used. The reaction conditions may be selected appropriately from the types of conditions typically used in the RPA method, or modified conditions thereof.

The LAMP method is a method in which amplification is conducted using a strand displacement reaction, using four types of primers (FIP primer, F3 primer, BIP primer, and B3 primer) combining six regions from the target nucleotide sequence. The F3 primer corresponds with the "forward primer that hybridizes with the 5' end region of the target nucleotide sequence" in the present invention, and the B3 primer corresponds with the "reverse primer that hybridizes with the 3' end region of the target nucleotide sequence" in the present invention. These primers can be designed, for example, using the LAMP method primer design assist software "PrimerExplorer" (manufactured by Eiken Chemical Co., Ltd.). The strand displacement DNA polymerase and the buffer and the like used in preparing the reaction mixture may be selected appropriately from among substances typically used in the LAMP method or modified products of these substances, and the reaction conditions may also be selected appropriately from the types of conditions typically used in the LAMP method, or modified conditions thereof.

The MDA method is a method that generally uses random primers, and synthesizes a single-stranded nucleic acid from the position where the primer is bound using Phi29 DNA polymerase. The Phi29 DNA polymerase itself has helicase-like activity, and even when encountering a double-stranded nucleic acid (for example, an association product of the template DNA and a primer) during the nucleic acid synthesis, can facilitate progression of the DNA synthesis reaction while unwinding the double strands. It is known that strands with a length of more than 70,000 bases can be synthesized in a reaction at 30°C for 12 hours. By using random primers or primers close to the target nucleotide sequence, the target nucleotide sequence can be amplified. The primers and DNA polymerase used, and the buffer and the like used in preparing the reaction mixture may be selected appropriately from among substances typically used in the MDA method or modified products of these substances, and the reaction conditions may also be selected appropriately from the types of conditions typically used in the MDA method, or modified conditions thereof.

The NASBA method is an isothermal nucleic acid amplification reaction that is conducted using RNA as a template, and using AMV reverse transcriptase, an RNase H, a T7 RNA polymerase, and two types of primers (an F primer, and an R primer having a T7 promoter sequence at the 5' side). First, the R primer (corresponding with the "reverse primer that hybridizes with the 3' end region of the target nucleotide sequence" in the present invention) is annealed to the template RNA, a cDNA is synthesized by the reverse transcriptase, and the RNA strand portion of the thus obtained RNA/DNA strand is digested by the RNase H. The F primer (corresponding with the "forward primer that hybridizes with the 5' end region of the target nucleotide sequence" in the present invention) is then annealed to the residual cDNA, a cDNA is synthesized by the reverse transcriptase, and using the obtained two-stranded cDNA as a template, a single-stranded antisense RNA is synthesized by a transcription reaction of the T7 RNA polymerase. The F primer is annealed to this single-stranded RNA, and following formation of an RNA/DNA strand by the reverse transcriptase, the RNA strand portion is digested by the RNase H. The R primer is annealed to the residual cDNA, a double-stranded cDNA is produced by the reverse transcriptase, and using this double-stranded cDNA as a template, a single-stranded antisense RNA is synthesized by a transcription reaction of the T7 RNA polymerase. By repeating this process, the single-stranded antisense RNA of the RNA that represents the target nucleic acid is amplified. The primers, reverse transcriptase, RNase H and T7 RNA polymerase used, and the buffer and the like used in preparing the reaction mixture may be selected appropriately from among substances typically used in the NASBA method or modified products of these substances, and the reaction conditions may also be selected appropriately from the types of conditions typically used in the NASBA method, or modified conditions thereof.

Reverse transcription is a reaction that uses an RNA as a template and synthesizes an antisense cDNA strand of the template RNA using a reverse transcriptase. By performing the RT-PCR method, which combines reverse transcription with PCR using the thus obtained cDNA as a template, the target RNA can be detected with high sensitivity. The primers, reverse transcriptase and DNA polymerase used, and the buffer and the like used in preparing the reaction mixture may be selected appropriately from among substances typically used in reverse transcription reactions or modified products of these substances.

There are no particular limitations on the reaction temperature during the RPA method, LAMP method, MDA method, NASBA method and reverse transcription reaction, provided the temperature falls within the temperature range in which the enzyme being used exhibits activity. Further, in an isothermal nucleic acid amplification reaction, the amount of the amplification product obtained depends on the reaction time. Accordingly, the reaction time is preferably selected appropriately in accordance with the amount of amplification product being targeted. For example, in the RPA method and the LAMP method, the reaction time may be set within a range from 5 minutes to 6 hours, and is preferably within a range from 5 minutes to 1 hour, and more preferably from 5 to 30 minutes. In the MDA method, the reaction time may be set within a range from 5 minutes to 32 hours, and is preferably within a range from 5 minutes to 24 hours, and more preferably from 5 minutes to 16 hours. In the NASBA method, the reaction time may be set within a range from 5 minutes to 6 hours, and is preferably within a range from 5 minutes to 3 hours, and more preferably from 5 minutes to 1.5 hours. In a reverse transcription reaction, the reaction time may be set within a range from 5 minutes to 3 hours, and is preferably within a range from 5 minutes to 1 hour, and more preferably from 5 to 30 minutes.

In the method for detecting a target nucleic acid according to the present invention, there are no particular limitations on the target region-specific binding molecule included in reaction system for the nucleic acid amplification reaction, provided the molecule binds specifically with the nucleic acid other than the target nucleic acid that can be amplified by the nucleic acid amplification reaction using the primer used in the target nucleic acid amplification, namely binds specifically with the non-target nucleic acid, thereby inhibiting nucleic acid amplification.

In those cases where the structural difference between the target nucleic acid and the non-target nucleic acid is a difference in nucleotide sequence, a molecule that specifically recognizes and binds to the nucleotide sequence of the target region of the non-target nucleic acid can be used as the target region-specific binding molecule. Examples of the molecule in those cases where the target nucleic acid is a DNA include complexes (CRISPR complexes) of a DNA strand cleavage activity-deficient Cas9 (dCas9) protein and a gRNA (guide RNA). Examples of the molecule in those cases where the target nucleic acid is an RNA include complexes (CRISPR complexes) of an RNA strand cleavage activity-deficient (dCas13a) protein of Cas13a that constitutes part of a CRISPR complex that binds with the RNA (Non-Patent Document 7) and a gRNA. In particular, a Cas13a/gRNA complex has been reported to be able to discern a difference in one base of an RNA (Non-Patent Document 8). Accordingly, by using a Cas13a/gRNA complex as the target region-specific binding molecule in the method for detecting a target nucleic acid according to the present invention, a target nucleic acid and a non-target nucleic acid that differ at only one base can be discriminated, enabling the target nucleic acid to be detected.

Examples of dCas9 proteins include proteins in which a mutation has been introduced into the nuclease domain of the Cas9 protein, thereby inactivating the nuclease activity while maintaining the DNA-binding ability. Specific examples of these proteins include a mutant derived from *Streptococcus pyogenes-derived* wild-type Cas9 protein, in which at least one point mutation has been introduced from among D10A in the RuvC nuclease domain (a point mutation in which aspartic acid 10 has been substituted with alanine) and H840A in the HNH nuclease domain (a point mutation in which histidine 840 has been substituted with alanine). Further, mutants of various types of Cas9 protein in which the two point mutations corresponding with D10A and H840A of *Streptococcus* pyogenes-derived Cas9 protein have been introduced may also be used. Commercially available proteins such as "EnGen Spy dCas9 (SNAP-tag)" (manufactured by New England Biolabs Ltd.) may also be used as the dCas9 protein.

Cas13a, which is also called C2c2, is a protein that constitutes a CRISPR complex that targets RNA, and has two HEPN domains (Higher Eukaryotes and Prokaryotes Nucleotide-binding domains) and one nuclease domain. Examples of dCas13a include proteins in which a mutation has been introduced into the nuclease domain of the Cas13a protein, thereby inactivating the nuclease activity while maintaining the RNA-binding ability. Specific examples of these proteins include a mutant derived from *Leptotrichia* wadei-derived wild-type Cas13a protein, in which at least one point mutation has been introduced from among R474A (a point mutation in which arginine 474 has been substituted with alanine) and R1046A (a point mutation in which the arginine 1046 has been substituted with alanine) in the nuclease domain (Non-Patent Document 10). Further, mutants of various types of Cas13a protein in which the two point mutations corresponding with R474A and R1046A of *Leptotrichia wadei-*derived Cas13a protein have been introduced may also be used.

The gRNA used in those cases where Cas9 is used includes crRNA (CRISPR RNA) and tracrRNA (trans-activating CRISPR RNA) derived from bacteria. Of these, the crRNA is a single-stranded RNA containing a region composed of a nucleotide sequence that is complementary to a portion of tracrRNA (a tracrRNA-binding region), and a region composed of a nucleotide sequence that is complementary to the nucleotide sequence capable of specific recognition and binding (namely, a target nucleic acid-binding region). The target nucleic acid-binding region is a DNA-binding region in those cases where the target nucleic acid is a DNA, and is an RNA-binding region in those cases where the target nucleic acid is an RNA. On the other hand, the tracrRNA is a single-stranded RNA having a region composed of a nucleotide sequence that is complementary to a portion of crRNA (a crRNA-binding region), and undergoes hybridization with crRNA in this region to form a hairpin structure. The crRNA and tracrRNA may be independent single-stranded RNAs, or may be a single-stranded RNA (sgRNA) in which the crRNA and tracrRNA are linked by a suitable RNA linker. These RNAs may be designed using the same methods as those typically used in genome editing. In contrast, Cas13a does not require tracrRNA, and crRNA alone can function as the gRNA. Accordingly, a single-stranded RNA may be used as the gRNA.

The target nucleic acid-binding region (DNA-binding region or RNA-binding region) in the gRNA used in the present invention is a region that binds with the target region in the non-target nucleic acid, and is a region composed of a nucleotide sequence that is complementary to the nucleotide sequence of the target region. By binding a CRISPR complex, namely, a complex of dCas9 protein and gRNA in the case where the target nucleic acid is a DNA, or a complex of dCas13a and gRNA in the case where the target nucleic acid is an RNA, to the target region of the non-target nucleic acid, primer annealing to the template nucleic acid and the nucleic acid extension reaction caused by the polymerase are inhibited, thereby inhibiting amplification of the non-target nucleic acid.

In those cases where Cas9 is used, the target nucleic acid-binding region (DNA-binding region) in the gRNA is typically selected as the nucleotide sequence of the region that is immediately followed by the PAM sequence. The PAM sequence is a sequence recognized by the dCas9 protein, and is determined on the basis of the Cas9 protein or the like being used. Although there are no particular limitations on the base length of the DNA-binding region to which the dCas9 protein binds, the base length is, for example, about 15 to 30 bases, and preferably within a range from 18 to 23 bases.

In those cases where Cas13a is used, the target nucleic acid-binding region (RNA-binding region) in the gRNA is typically selected as the nucleotide sequence of the region that is immediately followed by the PFS sequence. The PFS sequence is a sequence recognizes by the dCas13a protein, and is determined on the basis of the Cas13a protein or the like being used. Although there are no particular limitations on the base length of the RNA-binding region to which the dCas13a protein binds, the base length is, for example, about 15 to 40 bases, and preferably within a range from 20 to 30 bases.

Although there are no particular limitations on the amount of the dCas9 protein or dCas13a protein in the reaction mixture, the amount per 20 µL of the reaction mixture is, for example, set to not more than 200 ng, and is preferably within a range from 10 to 80 ng. Further, although there are no particular limitations on the amount of gRNA in the reaction mixture, the amount is, for example, set to not more than 50 nM, and is preferably within a range from 2.5 to 20 nM.

In addition to CRISPR complexes, molecules including proteins having a DNA sequence-specific binding ability or proteins having an RNA sequence-specific binding ability, which do not bind to the corresponding target region in the target nucleic acid, but bind specifically to the target region in the non-target nucleic acid, may also be used as the target region-specific binding molecule. Examples of these types of proteins include transcription regulators such as zinc finger proteins, TAL effectors (Transcription-Activator Like Effectors: TALE) and LexA proteins, which recognize and specifically bind to specific DNA sequences. Further examples include PPR (Pentatricopeptide Repeat) proteins and the like which recognize and bind specifically to specific RNA sequences.

In those cases where the non-target nucleic acid is a nucleic acid that differs from the target nucleic acid in terms of the modification state, and the target region in the non-target nucleic acid has a specific modification state which is not present in the corresponding target region of the target nucleic acid, a molecule that binds specifically to the modified nucleic acid can be used as the target region-specific binding molecule. For example, in those cases where the target region in the non-target nucleic acid is CpG methylated, whereas the corresponding target region in the target nucleic acid is not CpG methylated, a CpG methylated DNA-binding protein can be used as the target region-specific binding molecule. Conventional proteins such as proteins having an MBD (Methyl-CpG-binding Domain) (namely, MBD proteins) or antibodies that recognize methylated CpG may be used as appropriate as the CpG methylated DNA-binding protein. Although there are no particular limitations on the amount of the MBD2 protein in the reaction mixture, the amount per 20 µL of the reaction mixture is, for example, not more than 10 µg, and is preferably within a range from 0.05 to 2 µg.

In those cases where the non-target nucleic acid is a nucleic acid that differs from the target nucleic acid in terms of the modification state, and the target region in the non-target nucleic acid does not have a specific modification that is present in the corresponding target region of the target nucleic acid, a molecule that binds specifically to the unmodified nucleic acid and does not bind to the modified nucleic acid can be used as the target region-specific binding molecule. For example, in those cases where the target region in the non-target nucleic acid is not CpG methylated, whereas the corresponding target region in the target nucleic acid is CpG methylated, a protein that binds specifically to DNA that is not CpG methylated and does not bind to CpG methylated nucleic acids can be used as the target region-specific binding molecule. Examples of proteins that bind specifically to DNA that is not CpG methylated include CpG methyltransferase mutants having inactivated enzyme activity. Furthermore, if a substance that can act as a methyl group donor is not present in the reaction mixture, wild-type proteins of CpG methyltransferase can also be used as the above protein. Although there are no particular limitations on the amount in the reaction mixture of the protein that binds specifically to DNA that is not CpG methylated, the amount per 20 µL of the reaction mixture is, for example, not more than 10 µg, and is preferably within a range from 0.05 to 2 µg.

In those cases where two or more non-target nucleic acids exist, target region-specific binding molecules for each of the non-target nucleic acids may be added to the reaction system of the nucleic acid amplification reaction.

In the method for detecting a target nucleic acid according to the present invention, when an amplification product is obtained as a result of the nucleic acid amplification reaction, a judgement can be made that the target nucleic acid has been detected, meaning the target nucleic acid is contained in the nucleic acid test sample. On the other hand, when an amplification product is not obtained, a judgement can be made that the target nucleic acid has not been detected, meaning the target nucleic acid is not contained in the nucleic acid test sample. Because the target nucleic acid is detected as a positive signal for the amplification product, the target nucleic acid can be detected with good detection sensitivity.

The method for detecting a target nucleic acid according to the present invention is ideal, for example, for the detection and the like of gene mutations. The corresponding target region is specified as the mutation site targeted for detection, and the primers used in the nucleic acid amplification reaction are designed so that a nucleic acid amplification product of the region containing that mutation site is obtained when the target nucleic acid or non-target nucleic acid functions as the template. For example, the nucleic acid in which the mutation site is the mutated form (mutant nucleic acid) is specified as the target nucleic acid, the nucleic acid in which the mutation site is the wild form (wild-type nucleic acid) is specified as the non-target nucleic acid, and a molecule (excluding molecules composed solely of a nucleic acid) which binds specifically to the nucleic acid with a nucleotide sequence in which the mutation site is the wild form, but does not bind to the nucleic acid with a nucleotide sequence in which the mutation site is the mutated form is used as the target region-specific binding molecule. A nucleic acid amplification reaction is then conducted in the presence of this target region-specific binding molecule, under temperature conditions under which the binding activity of the target region-specific binding molecule to the wild-type nucleic acid is not lost. In those cases where the nucleic acid test sample contains the mutant nucleic acid, an amplification product is obtained, whereas in those cases where the nucleic acid test sample does not contain the mutant nucleic acid, no amplification product is obtained. The mutant nucleic acid that represents the target nucleic acid can be detected on the basis of the presence or absence of an amplification product. Accordingly, this method enables the detection of not only homozygous mutations, but also heterozygous mutations.

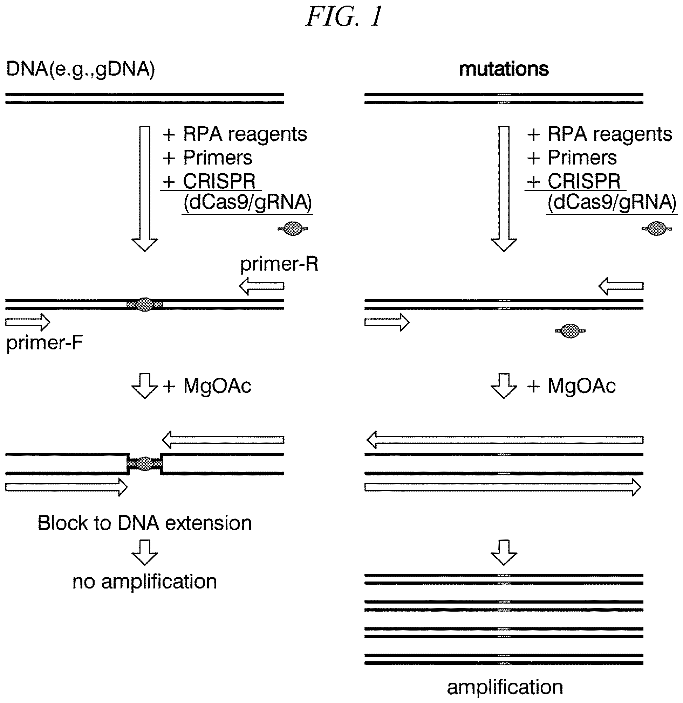

FIG. 1 is a diagram schematically illustrating the method in the case of detection of a gene mutation using a dCas9 protein and a gRNA as the target region-specific binding molecule. In the figure, the left side represents the amplification reaction using the wild-type nucleic acid as a template, and the right side represents the amplification reaction using the mutant nucleic acid as a template.

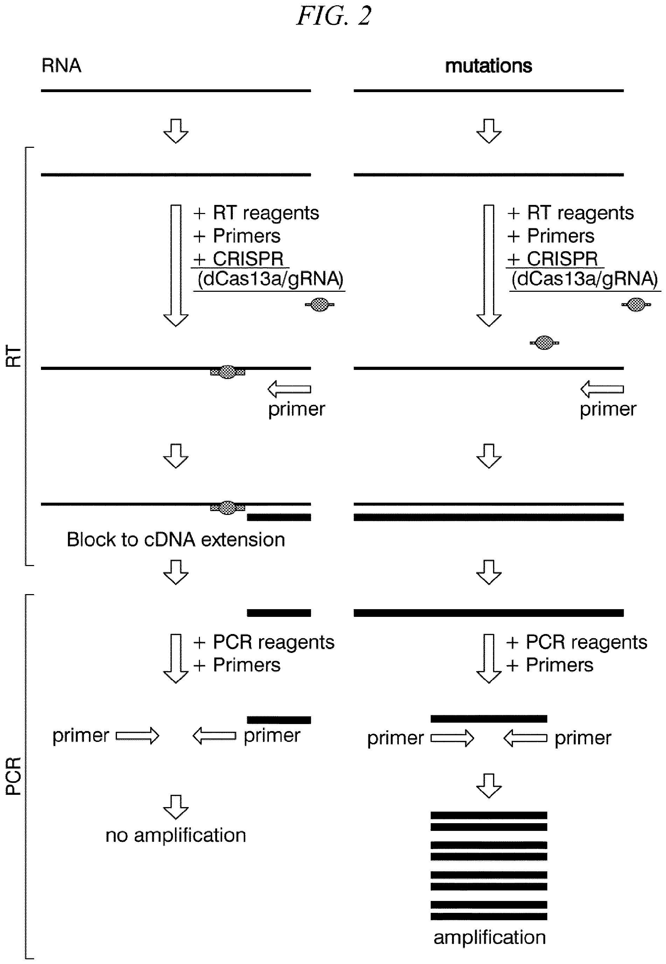

FIG. 2 is a diagram schematically illustrating the method in the case of detection of a gene mutation by RT-PCR with an RNA as the target nucleic acid. A dCas13a protein/gRNA complex that binds with the wild-type RNA was used as the target region-specific binding molecule. In the figure, the left side represents the amplification reaction using the mutant RNA as a template, and the right side represents the amplification reaction using the wild-type RNA as a template. Because the dCas13a protein/gRNA complex binds with the wild-type RNA, the cDNA extension caused by the reverse transcriptase is inhibited, meaning a PCR amplification product is not obtained. With the mutant RNA, the RT-PCR yields an amplification product.

The method for detecting a target nucleic acid according to the present invention is also ideal, for example, for the detection of gene polymorphism. The target region is specified as the polymorphic site targeted for detection, and the primers used in the nucleic acid amplification reaction are designed so that a nucleic acid amplification product of the region containing that polymorphic site is obtained when the target nucleic acid or non-target nucleic acid functions as the template. For example, the nucleic acid in which the polymorphic site is the target genotype is specified as the target nucleic acid, the nucleic acid in which the polymorphic site is a genotype other than the target genotype is specified as the non-target nucleic acid, and a molecule (excluding molecules composed solely of a nucleic acid) which binds specifically to the nucleic acid in which the polymorphic site is a genotype other than the target genotype is used as the target region-specific binding molecule. A nucleic acid amplification reaction is then conducted in the presence of this target region-specific binding molecule, under temperature conditions under which the binding activity of the target region-specific binding molecule to the non-target nucleic acid is not lost. In those cases where the provided nucleic acid test sample contains the targeted genotype nucleic acid to be detected, an amplification product is obtained, whereas in those cases where the nucleic acid test sample does not contain the targeted genotype nucleic acid, no amplification product is obtained. The gene polymorphism of the specific genotype that represents the target nucleic acid can be detected on the basis of the presence or absence of an amplification product. In a similar manner to above, this method also enables the detection of not only homozygous polymorphism, but also heterozygous polymorphism.

One method for analyzing DNA methylation is a method that utilizes a bisulfite treatment. By subjecting the DNA to be analyzed to a bisulfite treatment, using a reaction time that is suitable to ensure that methylated bases are not deaminated, whereas non-methylated bases are deaminated, and then conducting a nucleic acid amplification reaction with a polymerase, an amplification product is obtained in which the methylated bases are retained as the original bases, whereas the non-methylated bases are deaminated and replaced with different bases. For example, because a cytosine is converted to a uracil by deamination, in the amplification product, a methylated cytosine is a cytosine, whereas a non-methylated cytosine becomes a thymine.

In those cases where the methylation states of the corresponding target region and the target region differ, conducting a bisulfite treatment causes a difference in the bases to develop between the corresponding target region and the target region. Accordingly, by using a bisulfite-treated nucleic acid as the nucleic acid test sample, the method for detecting a target nucleic acid according to the present invention can be used to analyze whether or not a specific base in the nucleic acid is methylated. A nucleic acid in which the base that represents the analysis target is methylated may be used as the target nucleic acid, or a nucleic acid in which the base that represents the analysis target is not methylated may be used as the target nucleic acid.

For example, to analyze whether or not a specific cytosine is methylated, in the case where a bisulfite-treated nucleic acid in which the cytosine of the analysis target is methylated is used as the target nucleic acid, the partial region in the target nucleic acid containing the cytosine (methylated cytosine) of the analysis target is specified as the corresponding target region, and a bisulfite-treated nucleic acid in which the cytosine of the analysis target is not methylated is used as the non-target nucleic acid. In this case, the cytosine of the analysis target in the non-target nucleic acid is converted to a uracil by the bisulfite treatment. The partial region containing this uracil is specified as the target region. When the nucleic acid test sample contains the nucleic acid in which the cytosine of the analysis target is a methylated cytosine, conducting a nucleic acid amplification reaction in the presence of the target region-specific binding molecule yields a nucleic acid amplification product in which the base is a cytosine. However, when the cytosine of the analysis target is a non-methylated cytosine in all of the nucleic acids contained in the nucleic acid test sample, the nucleic acid amplification reaction conducted in the presence of the target region-specific binding molecule does not yield an amplification product.

In the case where a bisulfite-treated nucleic acid in which the cytosine of the analysis target is not methylated is used as the target nucleic acid, the partial region in the target nucleic acid containing the cytosine (non-methylated cytosine) of the analysis target is specified as the corresponding target region, and a bisulfite-treated nucleic acid in which the cytosine of the analysis target is methylated is used as the non-target nucleic acid. In this case, because the cytosine of the analysis target in the non-target nucleic acid is methylated, the cytosine is retained as a methylate cytosine even after the bisulfite treatment. The partial region containing this methylated cytosine is specified as the target region. When the nucleic acid test sample contains the nucleic acid in which the cytosine of the analysis target is a non-methylated cytosine, conducting a nucleic acid amplification reaction in the presence of the target region-specific binding molecule yields a nucleic acid amplification product in which the base is a thymine. However, when the cytosine of the analysis target is a methylated cytosine in all of the nucleic acids contained in the nucleic acid test sample, the nucleic acid amplification reaction conducted in the presence of the target region-specific binding molecule does not yield an amplification product.

In each of these methods, a CRISPR complex or the like in which, for example, the target nucleic acid-binding region (DNA-binding region or RNA-binding region) of the gRNA has a nucleotide sequence that is complementary to the target region can be used as the target region-specific binding molecule that binds specifically to the target region but does not bind to the corresponding target region.

The amplification product of the target nucleic acid obtained from the nucleic acid amplification reaction can be detected using any of various methods typically used for the detection of amplification products of nucleic acid amplification reactions such as PCR. Examples of the detection method include methods in which bands that have been separated by agarose gel electrophoresis are stained using ethidium bromide or the like, intercalator methods, and melting curve analysis methods. Intercalator methods are methods that utilize the emission of fluorescence that occurs when a fluorescent intercalator such as SYBR Green binds to the produced double-stranded DNA, with the fluorescent intensity increasing as the amount of the amplification product increases. By irradiating an excitation light onto the reaction mixture and measuring the fluorescent intensity, the production amount of the amplification product can be monitored. Further, in those cases where a primer that has been modified with a labeling substance is used, detection may be conducted using the labeling substance as an indicator. In those cases where a primer that has been labeled with a fluorescent substance is used, the amplification product can be detected by removing the unreacted primer from the reaction mixture following the nucleic acid amplification reaction by column chromatography or the like, and then measuring the fluorescent intensity.

By combining the reagents and the like used in the method for detecting a target nucleic acid according to the present invention into a kit, the method can be conducted more simply. Among the various forms of the method for detecting a target nucleic acid according to the present invention, in the case of a method that uses a dCas9 protein and a gRNA as the target region-specific binding molecule, the kit preferably includes a primer that hybridizes with the target nucleic acid and the non-target nucleic acid, the dCas9 protein, and the gRNA. Among the various forms of the method for detecting a target nucleic acid according to the present invention, in the case of a method that uses a dCas13a protein and a gRNA as the target region-specific binding molecule, the kit preferably includes a primer that hybridizes with the target nucleic acid and the non-target nucleic acid, the dCas13a protein, and the gRNA. Further, among the various forms of the method for detecting a target nucleic acid according to the present invention, in the case of a method that uses a CpG methylated DNA-binding protein as the target region-specific binding molecule, the kit preferably includes a primer that hybridizes with both the target nucleic acid and the non-target nucleic acid, and the CpG methylated DNA-binding protein. Furthermore, in the case of a method that uses a transcription regulator as the target region-specific binding molecule, the kit preferably includes a primer that hybridizes with both the target nucleic acid and the non-target nucleic acid, and the transcription regulator.

The target nucleic acid detection kit according to the present invention preferably also includes an enzyme for conducting the nucleic acid amplification reaction, a concentrated buffer for preparing the reaction mixture, and dNTP or the like. For example, in the case of a kit used in a method in which the nucleic acid amplification reaction is conducted by the RPA method, the kit preferably also includes a recombinase, SSB, and a DNA polymerase.

The target nucleic acid detection kit according to the present invention preferably also includes documentation describing the protocol for using the kit to implement the method for detecting a target nucleic acid according to the present invention. The protocol may be recorded on the surface of the container that houses the kit.

### <Method for Detecting Nucleic Acid-Bonding Molecule>

By binding some form of substance to a nucleic acid that functions as a template, and utilizing reactions that impede primer annealing to the template nucleic acid and the nucleic acid extension reaction caused by a polymerase, a nucleic acid-binding molecule (a molecule that has a nucleic acid-binding ability) can be detected.

In other words, a method for detecting a nucleic acid-binding molecule according to the present invention involves conducting a nucleic acid amplification reaction using a test sample, a nucleic acid, and a primer that hybridizes with the nucleic acid. In those cases where the test sample contains a nucleic acid-binding molecule that binds with the nucleic acid used as a template, the nucleic acid amplification reaction does not yield an amplification product. In contrast, in those cases where the test sample does not contain a nucleic acid-binding molecule that binds with the nucleic acid used, the nucleic acid amplification reaction yields an amplification product. In other words, the presence of the nucleic acid-binding molecule in the test sample can be detected on the basis of the amount of the nucleic acid amplification product following the nucleic acid amplification reaction.

There are no particular limitations on the test sample supplied to the method for detecting a nucleic acid-binding molecule according to the present invention, provided there is an expectation that the sample may contain the nucleic acid-binding molecule. For example, tissue or cell extracts (lysates) from animals or plants, cell extracts of cultured cells, samples collected from the natural world such as soils, and crudely purified products of such natural samples may be used. The tissue or cell extracts can be prepared using general methods.

Although there are no particular limitations on the nucleic acid-binding molecule detected in the method for detecting a nucleic acid-binding molecule according to the present invention, a peptide, protein, or low-molecular weight compound is preferred, although a complex of a nucleic acid and another molecule such as a protein may also be detected. Further, the nucleic acid-binding molecule may be an unidentified substance. For example, by using a sample containing various substances as the test sample, such as a cell extract or nuclear extract of various cells, a determination can be made as to whether the test sample contains a nucleic acid-binding molecule. Furthermore, there are no particular limitations on the nucleic acid-binding ability of the nucleic acid-binding molecule detected in the method for detecting a nucleic acid-binding molecule according to the present invention, and the molecule may be a nucleotide sequence-specific nucleic acid-binding molecule that recognizes and binds specifically to a specific nucleotide sequence, a nucleic acid-binding molecule that recognizes and binds to a specific modification state, or a nucleic acid-binding molecule that binds generally to a wide range of nucleic acids, regardless of the nucleotide sequence or modification state.

The nucleic acid used in the method for detecting a nucleic acid-binding molecule according to the present invention is used as the template for the nucleic acid amplification reaction, and although there are no particular limitations on the nucleic acid, provided it is capable of functioning as the template for the nucleic acid amplification reaction, a DNA or RNA is preferred. The nucleic acid may have a specified nucleotide sequence, or may be a nucleic acid for which the nucleotide sequence is not specified. Further, the nucleic acid may be composed of a single nucleic acid, or may be a mixture of a wide variety of nucleic acids, such as a DNA that has been extracted from cells and purified. For example, in the case of detection of a nucleic acid-binding molecule that binds to nucleic acids without recognizing the nucleotide sequence, the nucleic acid used as the template may be a nucleic acid for which the nucleotide sequence is unknown or a mixture containing a wide variety of nucleic acids.

For example, in the case where a sequence-specific nucleic acid-binding molecule that recognizes and binds to a specified nucleotide sequence is to be detected, a nucleic acid having the specified nucleotide sequence is used as the template, and in order to ensure that a region containing the specified nucleotide sequence in the template nucleic acid is amplified in the nucleic acid amplification reaction, a primer that has been designed to hybridize with the upstream side (5' side) from the specified nucleotide sequence in the template nucleic acid is used. The primer can be designed and synthesized using general methods based on the nucleotide sequence of the nucleic acid used as the template.

In the case where a nucleic acid-binding molecule that binds specifically to a nucleic acid in a specific modification state is to be detected, the nucleic acid in a specific modification state is used as a template. Examples of the modification state include methylation, fluorination, phosphorothioation, phosphorodithioation, sugar addition, PEG (polyethylene glycol) addition, and peptide addition.

Examples of the nucleic acid amplification reaction conducted in the method for detecting a nucleic acid-binding molecule according to the present invention include the same nucleic acid amplification reactions as those conducted in the above method for detecting a target nucleic acid according to the present invention. In those cases where the nucleic acid-binding molecule targeted for detection is a molecule of comparatively low heat resistance such as a protein or the like, the nucleic acid amplification reaction is preferably conducted under temperature conditions of 65°C or lower. In contrast, in the case of detection of a heat-resistant nucleic acid-binding molecule, the nucleic acid amplification reaction may be conducted in a temperature range exceeding 65°C but not more than 100°C using a heat-resistant polymerase. Further, the nucleic acid amplification reaction conducted in the method for detecting a nucleic acid-binding molecule according to the present invention is preferably a nucleic acid amplification reaction that is conducted under isothermal conditions such as the RPA method, LAMP method, MDA method, or NASBA method or the like, as this negates the necessity for a thermal cycler that is required for conducting temperature control in PCR.

For example, the nucleic acid amplification reaction is conducted using the test sample, a nucleic acid, and a primer that hybridizes with that nucleic acid, and the amount of nucleic acid amplification product obtained is compared with the amount of nucleic acid amplification product obtained when the nucleic acid amplification reaction is conducted under the same conditions but with the exception of not adding the test sample. In those cases where the test sample contains a nucleic acid-binding molecule that binds to the nucleic acid used as the template, the amount of the nucleic acid amplification product obtained in the presence of the test sample will be less than the amount of the nucleic acid amplification product obtained in the absence of the test sample. In contrast, if the amount of the nucleic acid amplification product obtained in the presence of test sample is similar to or greater than the amount of the nucleic acid amplification product obtained in the absence of the test sample, then the test sample does not contain a nucleic acid-binding molecule that binds to the nucleic acid used as a template.

By combining the reagents and the like used in the method for detecting a nucleic acid-binding molecule according to the present invention into a kit, the method can be conducted more simply. For example, the nucleic acid-binding molecule detection kit for using this method preferably includes a nucleic acid that is used as the template, and a primer that hybridizes with the nucleic acid. In addition, the kit preferably also includes an enzyme for the nucleic acid amplification reaction, a concentrated buffer for preparing the reaction mixture, and dNTP or the like. For example, in the case of a kit used in a method in which the nucleic acid amplification reaction is conducted by the RPA method, the kit preferably also includes a recombinase, SSB, and a DNA polymerase.

The nucleic acid-binding molecule detection kit according to the present invention preferably also includes documentation describing the protocol for using the kit to implement the method for detecting a nucleic acid-binding molecule according to the present invention. The protocol may be recorded on the surface of the container that houses the kit.

### <Method for Evaluating Nucleic Acid-Binding Ability>

By binding some form of substance to a nucleic acid that functions as a template, and utilizing reactions that impede primer annealing to the template nucleic acid and the nucleic acid extension reaction caused by a polymerase, the nucleic acid-binding ability of a nucleic acid-binding can be evaluated.

In other words, a method for evaluating nucleic acid-binding ability according to the present invention involves conducting a nucleic acid amplification reaction using a test substance, a nucleic acid that evaluates the nucleic acid-binding ability of that test substance, and a primer that hybridizes with the nucleic acid. In those cases where the test substance has binding ability to the nucleic acid used as a template, the nucleic acid amplification reaction does not yield an amplification product. In contrast, in those cases where the test substance does not have binding ability to the nucleic acid, the nucleic acid amplification reaction yields an amplification product. In other words, the binding ability of the test substance to the nucleic acid used as a template can be evaluated on the basis of the amount of the nucleic acid amplification product following the nucleic acid amplification reaction.

There are no particular limitations on the test substance that represents the evaluation target in the method for evaluating nucleic acid-binding ability according to the present invention, but a peptide, protein, or low-molecular weight compound is preferred. Alternatively, a complex of a nucleic acid and another molecule such as a protein may also be used. Further, in addition to purified substances, the test substance may also be a composition that also contains substances other than the test substance. Moreover, the test substance may be an unidentified substance. For example, a substance containing various substances, such as a cell extract or nuclear extract of various cells, may also be used as the test substance.

There are no particular limitations on the nucleic acid-binding ability evaluated in the method for evaluating nucleic acid-binding ability according to the present invention, and the binding ability may be a sequence-specific nucleic acid-binding ability that recognizes and binds to a specific nucleotide sequence, a nucleic acid-binding ability that recognizes and binds to a specific modification state, or a nucleic acid-binding ability that binds generally to a wide range of nucleic acids, regardless of the nucleotide sequence or modification state.

The nucleic acid used in the method for evaluating nucleic acid-binding ability according to the present invention is the target nucleic acid used for evaluating whether or not the test substance has nucleic acid-binding ability, and although there are no particular limitations on the nucleic acid, provided it is capable of functioning as the template for the nucleic acid amplification reaction, a DNA or RNA is preferred. The nucleic acid may have a specified nucleotide sequence, or may be a nucleic acid for which the nucleotide sequence is not specified. Further, the nucleic acid may be composed of a single nucleic acid, or may be a mixture of a wide variety of nucleic acids.

For example, in the case where a sequence-specific nucleic acid-binding ability that recognizes and binds to a specified nucleotide sequence is to be evaluated, a nucleic acid having the specified nucleotide sequence is used as the template, and in order to ensure that a region containing the specified nucleotide sequence in the template nucleic acid is amplified in the nucleic acid amplification reaction, a primer that has been designed to hybridize with the upstream side (5' side) from the specified nucleotide sequence in the template nucleic acid is used. The primer can be designed and synthesized using typical methods based on the nucleotide sequence of the nucleic acid used as the template.

In the case where a nucleic acid-binding ability that binds specifically to a nucleic acid in a specific modification state is to be evaluated, the nucleic acid in a specific modification state is used as a template. Examples of the modification state include methylation, fluorination, phosphorothioation, phosphorodithioation, sugar addition, PEG (polyethylene glycol) addition, and peptide addition.

Examples of the nucleic acid amplification reaction conducted in the method for evaluating nucleic acid-binding ability according to the present invention include the same nucleic acid amplification reactions as those conducted in the above method for detecting a target nucleic acid according to the present invention. In those cases where the target substance is a molecule of comparatively low heat resistance such as a protein or the like, the nucleic acid amplification reaction is preferably conducted under temperature conditions of 65°C or lower. In contrast, in the case where the test substance is a molecule having high heat resistance, and the nucleic acid-binding ability under high temperature conditions is to be evaluated, the nucleic acid amplification reaction may be conducted in a temperature range exceeding 65°C but not more than 100°C using a heat-resistant polymerase. Further, the nucleic acid amplification reaction conducted in the method for evaluating nucleic acid-binding ability according to the present invention is preferably a nucleic acid amplification reaction that is conducted under isothermal conditions such as the RPA method, LAMP method, MDA method, or NASBA method or the like, as this negates the necessity for a thermal cycler that is required for conducting temperature control in PCR.

For example, the nucleic acid amplification reaction is conducted using the test substance, a nucleic acid, and a primer that hybridizes with that nucleic acid, and the amount of nucleic acid amplification product obtained is compared with the amount of amplification product obtained when the nucleic acid amplification reaction is conducted under the same conditions but with the exception of not adding the test substance. In those cases where the test substance has nucleic acid-binding ability to the template nucleic acid under the temperature conditions and the like under which the nucleic acid amplification reaction is conducted, the amount of the nucleic acid amplification product obtained in the presence of the test substance will be less than the amount of the nucleic acid amplification product obtained in the absence of the test substance. In other words, in those cases where the amount of the nucleic acid amplification product obtained in the presence of the test substance is less than the amount of the nucleic acid amplification product obtained in the absence of the test substance, the test substance is evaluated as having nucleic acid-binding ability to the nucleic acid used as a template under the temperature conditions and the like under which the nucleic acid amplification reaction was conducted. In contrast, if the amount of the nucleic acid amplification product obtained in the presence of test substance is similar to or greater than the amount of the nucleic acid amplification product obtained in the absence of the test substance, then the test substance is evaluated as not having nucleic acid-binding ability to the nucleic acid used as a template.

By combining the reagents and the like used in the method for evaluating nucleic acid-binding ability according to the present invention into a kit, the method can be conducted more simply. For example, the nucleic acid-binding ability evaluation kit for using this method preferably includes a target nucleic acid that evaluates the nucleic acid-binding ability of a test substance, and a primer that hybridizes with the nucleic acid. In addition, the kit preferably also includes an enzyme for the nucleic acid amplification reaction, a concentrated buffer for preparing the reaction mixture, and dNTP or the like. For example, in the case of a kit used in a method in which the nucleic acid amplification reaction is conducted by the RPA method, the kit preferably also includes a recombinase, SSB, and a DNA polymerase.

The nucleic acid-binding ability evaluation kit according to the present invention preferably also includes documentation describing the protocol for using the kit to implement the method for evaluating nucleic acid-binding ability according to the present invention. The protocol may be recorded on the surface of the container that houses the kit.

### EXAMPLES

The present invention is described below in further detail based on a series of examples, but the present invention is not limited by these examples.

### [RNA and Primers]

The RNA used in the tests described below were RNA chemically synthesized by FASMAC Co., Ltd. The various RNA used are shown in Table 1.

**[Table 1]**

| RNA | Sequence (5' → 3') | SEQ ID NO: |
|---|---|---|
| crRNA_KRAS | guaguuggagcugguggcguguuuuagagcuaugcuguuuug | 1 |
| crRNA_KRAS#2 | cuugugguaguuggagcuggguuuuagagcuaugcuguuuug | 2 |
| crRNA_hp16_Gx5#2 | acggccgcggcccgggggucguuuuagagcuaugcuguuuug | 3 |
| crRNA_KRAS_mut | guaguuggagcuggaggcguguuuuagagcuaugcuguuuug | 4 |
| crRNA_mid2 | caccuccucuacccgaccccguuuuagagcuaugcuguuu | 5 |
| tracrRNA | aaacagcauagcaaguuaaaauaaggcuaguccguuaucaacuu gaaaaaguggcaccgagucggugcu | 6 |

The primers used in the tests described below were primers chemically synthesized by Eurofins Scientific S.E. The primers used are shown in Table 2.

**[Table 2]**

| Primer | Sequence (5' → 3') | SEQ ID NO: |
|---|---|---|
| KRAS-RPA-G12-F | tagtgtattaaccttatgtgtgacatgttctaat | 7 |
| KRAS-RPA-G12-R | aaacaagatttacctctattgttggatcatattc | 8 |
| p16-RPA-F2 | ggcggcggggagcagcatggagccttcggctgac | 9 |
| p16-RPA-R2 | ctacccacctggatcggcctccgaccgtaactat | 10 |
| p14-RPA-F | gtcccagtctgcagttaagggggcaggagt | 11 |
| p14-RPA-R | gggcctttcctacctggtcttctaggaa | 12 |
| p16-RPA-F | gaggaagaaagaggaggggctggctggtcacc | 13 |
| p16-RPA-R | ctgcagaccctctacccacctggatcggcctc | 14 |
| p14ARF-CpG_island-F | gtgggtcccagtctgcagttaag | 15 |
| p14ARF-CpG_island-R | acttttcgagggcctttcctac | 16 |
| Pax5-LexA-RPA-F | gcatcagtcgcccttcgcctccttctctcg | 17 |
| Pax5-LexA-RPA-R | gcgagggcggaacgtgactttgccctgcgg | 18 |

### [Production of gRNA]

By mixing 1 µL of 10 µM crRNA, 1 µL of 10 µM tracrRNA, and 2 µL of nuclease-free water, and incubating the mixture at 98°C for 2 minutes, a gRNA (crRNA/tracr RNA complex) was formed.

### [RPA Reaction]

An RPA reaction was conducted in the manner described below. First, 29.5 µL of rehydration buffer, 2.5 µL of 10 µM forward primer, and 2.5 µL of 10 µM reverse primer were added to and mixed with one tube (containing lyophilized reagent) of an RPA reagent (product name: TwistAmp (a registered trademark) Basic kit, manufactured by TwistDx Ltd.). Next, 13.6 µL of the thus prepared solution was transferred to a separate tube, and 20 ng of a template DNA and nuclease-free water were added, thus preparing 19 µL of an RPA reaction preparatory solution. Subsequently, 1 µL of 280 mM MgOAc solution was added to this RPA reaction preparatory solution, and the resulting mixture was incubated at 37°C for 30 minutes to conduct an RPA reaction.

An RPA reaction using a dCas9 protein and gRNA was conducted in the manner described below. The dCas9 used was a *Streptococcus pyogenes-derived* Cas9 having point mutations introduced at D10A and H840A, and was synthesized by Sysmex Corporation (ProCube, production number: 14M_029). First, 0.8 µL of gRNA, 0.4 µg of dCas9 protein and nuclease-free water were mixed to prepare 10 µL, and this solution was used as a CRISPR solution. During the RPA reaction, 1 µL of the CRISPR solution was added to the RPA reaction preparatory solution prepared above (following addition, the solution contained 40 ng of dCas9 protein and 10 nM gRNA), and the resulting solution was incubated at 37°C for 5 minutes. Subsequently, 1 µL of a 280 mM MgOAc solution was added to the reaction mixture, and the resulting mixture was incubated at 37°C for 30 minutes to conduct the RPA reaction.

Following completion of the reaction, a "PCR/Gel DNA purification kit" (manufactured by Nippon Genetics Co., Ltd.) was used to purify the nucleic acids from each of the reaction mixtures. The purified products were analyzed by electrophoresis using an agarose gel containing "SYBR (a registered trademark) Safe DNA Gel Stain" (manufactured by Thermo Fisher Scientific Inc.).

### [Example 1]

Two types of gRNA were produced for the *KRAS* gene of cells of the cultured cell line 293T, each of the produced gRNAs was added to a reaction system with dCas9 respectively, and an RPA reaction was conducted.

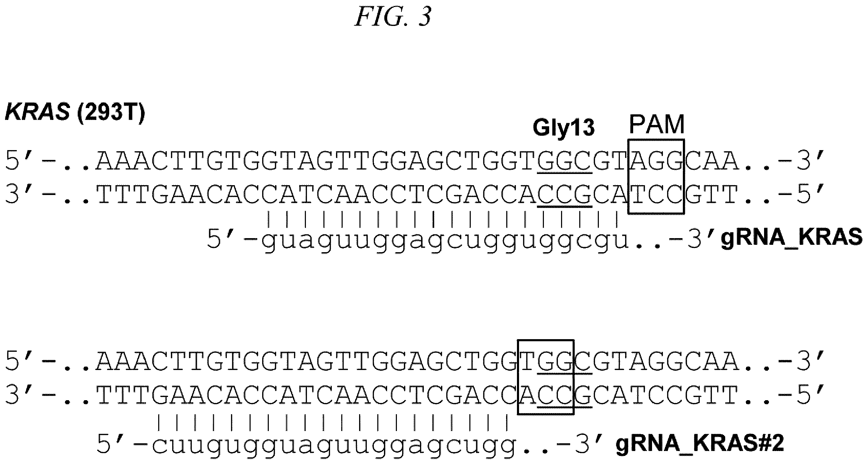

Genome DNA (293T gDNA) extracted from the 293T cells and purified was used as a template, and gRNA_KRAS (SEQ ID NOS: 1 and 6) and gRNA_KRAS#2 (SEQ ID NOS: 2 and 6) were used as gRNA. A forward primer (SEQ ID NO: 7) and a reverse primer (SEQ ID NO: 8) for amplifying the region, within the *KRAS* gene genome DNA, containing the gRNA_KRAS and gRNA_KRAS#2 target regions were used as a primer set. A partial region of the *KRAS* gene of the 293T cells, and the sequence portions complementary to the two types of gRNA used are shown in FIG. 3. Moreover, gRNA (gRNA_p16_Gx5#2, SEQ ID NOS: 3 and 6) for the *CDKN2A (p16)* gene was used as a control.

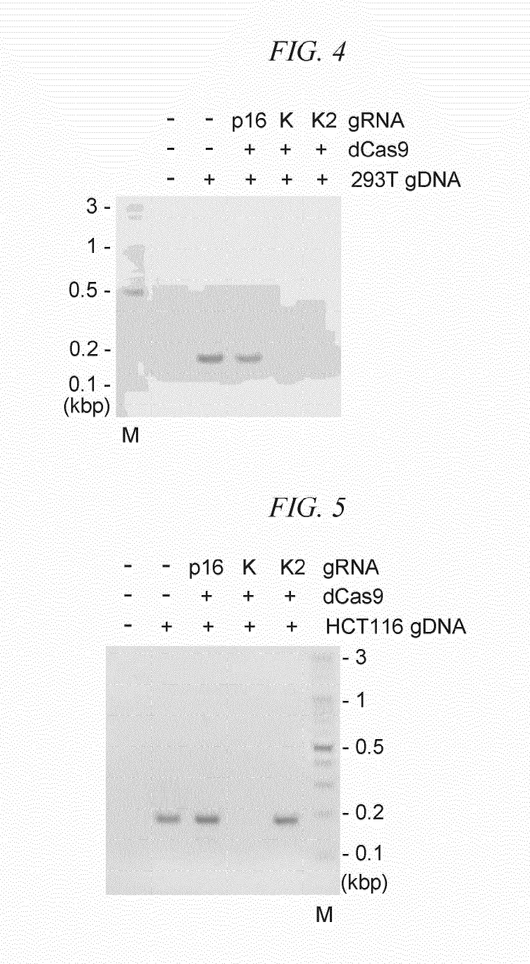

The electrophoresis results are shown in FIG. 4. In the reaction mixture to which gRNA_p16_Gx5#2 had been added, in a similar manner to that observed for the reaction mixture to which gRNA and dCas9 had not been added, an amplification product band was detected. In contrast, in the case of the reaction mixtures to which gRNA_KRAS or gRNA_KRAS#2 had been added, in both cases, an amplification product band was not detected, indicating that nucleic acid amplification of the *KRAS* gene by the forward primer and reverse primer had been inhibited. These results confirmed that by including, in the reaction mixture, gRNA and dCas9 for the DNA for which it is desirable to inhibit nucleic acid amplification, nucleic acid amplification by the RPA reaction could be inhibited.

### [Example 2]

In the *KRAS* gene of the cultured cell line HCT116, a single base substitution mutation (GGC → GAC) occurs in only one allele, with glycine 13 substituted with aspartic acid. As a result of this single base substitution, the PAM sequence (TGG) in the gRNA_KRAS#2 shown in FIG. 3 becomes TGA, and is no longer a PAM sequence (NGG).

With the exception of using genome DNA (HCT116 gDNA) extracted from the HCT116 cells and purified as the template nucleic acid, RPA reactions were conducted in the same manner as Example 1, and following purification, electrophoresis was conducted.

The electrophoresis results are shown in FIG. 5. In a similar manner to Example 1, which used 293T gDNA having only the wild-type *KRAS* gene as a template, a nucleic acid amplification product was confirmed in the reaction mixture to which the gRNA_p16_Gx5#2 had been added, but no amplification product was detected in the reaction mixture to which the gRNA_KRAS had been added. In other words, the gRNA_KRAS functioned as gRNA even though there was a single base mismatch between the gRNA and the template nucleic acid. On the other hand, unlike Example 1, a nucleic acid amplification product was also confirmed in the reaction mixture to which the gRNA_KRAS#2 had been added.

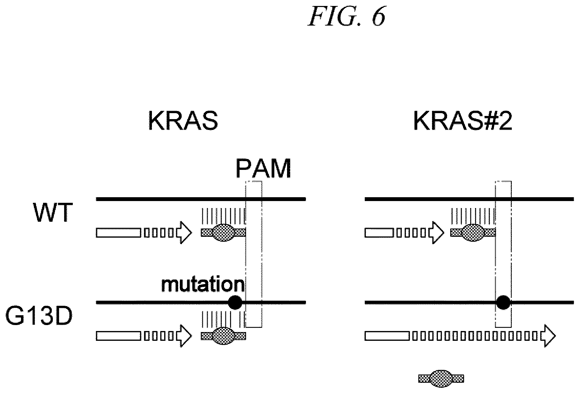

Confirmation of the nucleotide sequences of the amplification products obtained in the various reaction mixtures by sequence analysis revealed that the amplification products in the reaction mixture containing no added gRNA and the reaction mixture to which gRNA_p16_Gx5#2 had been added included both the wild-type *KRAS* and the mutant *KRAS (G13D)* which were originally included in the HCT116 gDNA. In contrast, the amplification product in the reaction mixture to which the gRNA_KRAS#2 had been added contained only the mutant *KRAS (G13D),* and no nucleic acid amplification of the wild-type *KRAS* was confirmed. FIG. 6 schematically illustrates the RPA reaction that occurred in the reaction mixture to which the gRNA_KRAS#2 had been added. In the case of the wild-type *KRAS*, because the gRNA_KRAS#2 binds together with the dCas9 in the vicinity of the PAM sequence, the amplification reaction by the DNA polymerase was inhibited, but in the case of the mutant *KRAS,* because there is no PAM sequence in the vicinity of the region to which gRNA_KRAS#2 is programmed to bind, the dCas9 was not recruited, and the amplification reaction proceeded normally. In other words, it was confirmed that by designing the gRNA so that the mutation site falls in the PAM sequence, a single base mutation could be detected extremely precisely.

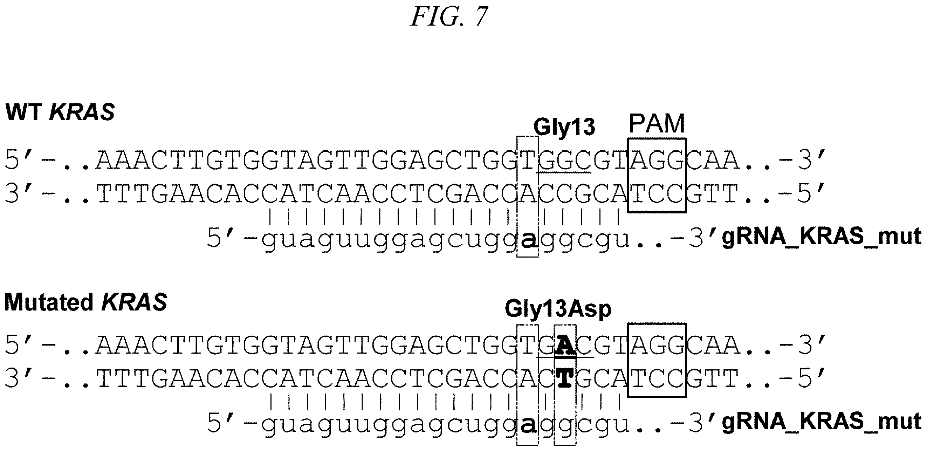

Next, as illustrated in FIG. 7, a single base substitution mutation was introduced into gRNA_KRAS. This gRNA_KRAS_mut (SEQ ID NOS: 4 and 6) differs from the wild-type *KRAS* by a single base, and differs from the mutant *KRAS (G13D)* by two bases. Using the HCT116 gDNA or 293T gDNA as a template nucleic acid, but with the exception of using gRNA_KRAS_mut as the gRNA, RPA reactions were conducted in the same manner as Example 1, and following purification, electrophoresis was conducted.

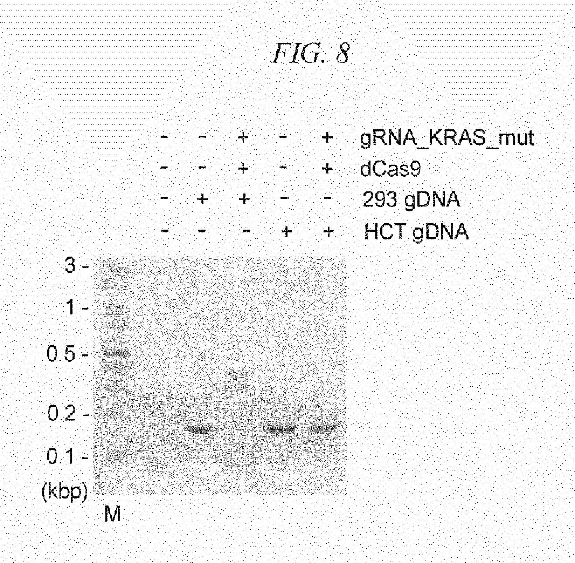

The electrophoresis results are shown in FIG. 8. The results revealed that in the reaction mixtures to which the 293T gDNA had been added, nucleic acid amplification was inhibited by the gRNA-KRAS_mut. In the reaction mixtures to which the HCT116 gDNA had been added, an amplification product band was detected even in the case where the gRNA_KRAS_mut had been added. Confirmation of the nucleotide sequences of the amplification products in the reaction mixtures to which the HCT116 gDNA had been added revealed that when gRNA_KRAS_mut was not added, the product included both the wild-type *KRAS* and the mutant *KRAS (G13D).* In contrast, when the gRNA_KRAS_mut was added, only the mutant *KRAS (G13D)* was included. In other words, the gRNA_KRAS_mut functioned as gRNA even when there was a single base mismatch between the gRNA and the template nucleic acid, and the nucleic acid amplification by the RPA reaction could be inhibited, but when there was a two-base mismatch between the gRNA and the template nucleic acid, the gRNA_KRAS_mut could not function as gRNA, and the nucleic acid amplification by the RPA reaction proceeded normally. These results confirmed that even in those cases where the mutation does not exist on the PAM sequence, by artificially introducing a mutation into the gRNA and increasing the mismatch with the target nucleotide sequence, a single base mutation could be distinguished.

### [Example 3]

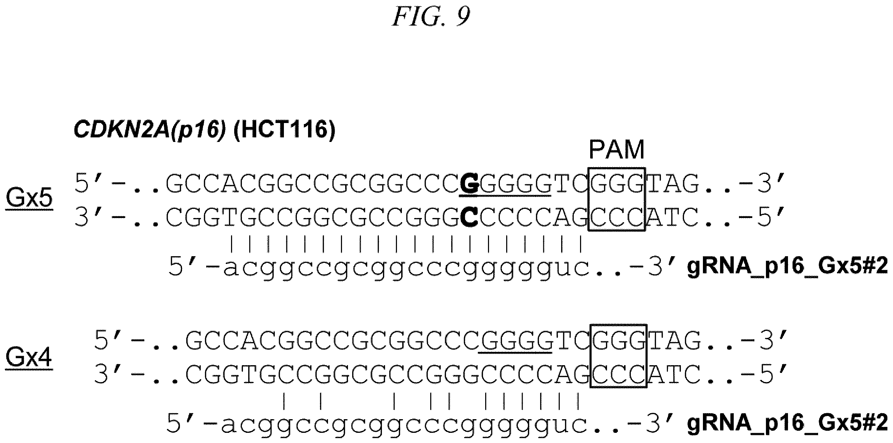

As illustrated in FIG. 9, in the *CDKN2A (p16)* gene of HCT116 cells, a single G is inserted in only one allele (Gx5). As a result, gRNA_p16_Gx5#2 has the same nucleotide sequence as the Gx5 allele, but does not match the other allele (Gx4).

With the exceptions of using genome DNA (HCT116 gDNA) extracted from HCT116 cells and purified as the template nucleic acid, using a forward primer (SEQ ID NO: 9) and a reverse primer (SEQ ID NO: 10) for amplifying the region, within the *CDKN2A (p16)* gene genome DNA, containing the gRNA_p16_Gx5#2 target region as a primer set, and using either gRNA_p16_Gx5#2 or gRNA_KRAS as the gRNA, RPA reactions were conducted in the same manner as Example 1, and following purification, electrophoresis was conducted.

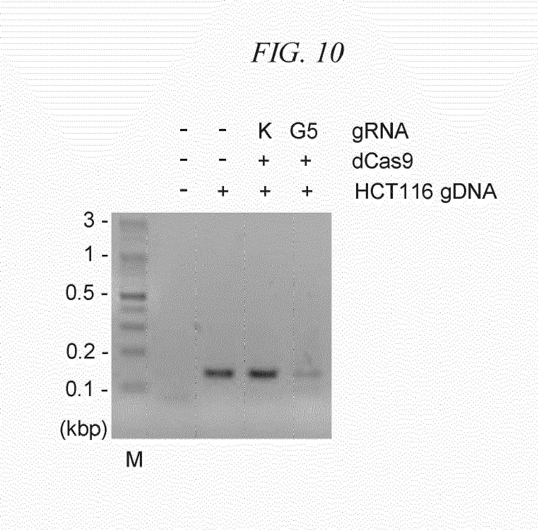

The electrophoresis results are shown in FIG. 10. Even in the reaction mixture containing the added gRNA_p16_Gx5#2, an amplification product was confirmed, although the amount was less than the amount of amplification product detected in the reaction mixture containing the added gRNA_KRAS or the reaction mixture containing no added gRNA.

Confirmation of the nucleotide sequences of the amplification products obtained in the various reaction mixtures by sequence analysis revealed that the amplification products in the reaction mixture containing no added gRNA and the reaction mixture to which gRNA_KRAS had been added included both the Gx5-type p16 and the Gx4-type p16 which were originally included in the HCT116 gDNA. In contrast, the amplification product in the reaction mixture to which the gRNA_p16_Gx5#2 had been added contained only the Gx4-type p16, and no nucleic acid amplification of the Gx5-type p16 was confirmed. This indicates that in the Gx5-type p16 sequence, the gRNA_p16_Gx5#2 is recruited together with the dCas9, inhibiting the amplification reaction by the DNA polymerase, whereas in the Gx4-type p16 sequence, the dCas9 is not recruited, and the amplification reaction proceeds normally. In other words, by using the method for detecting a target nucleic acid according to the present invention, it was confirmed that mutations having a single base insertion or deletion could be detected extremely precisely.

### [Example 4]

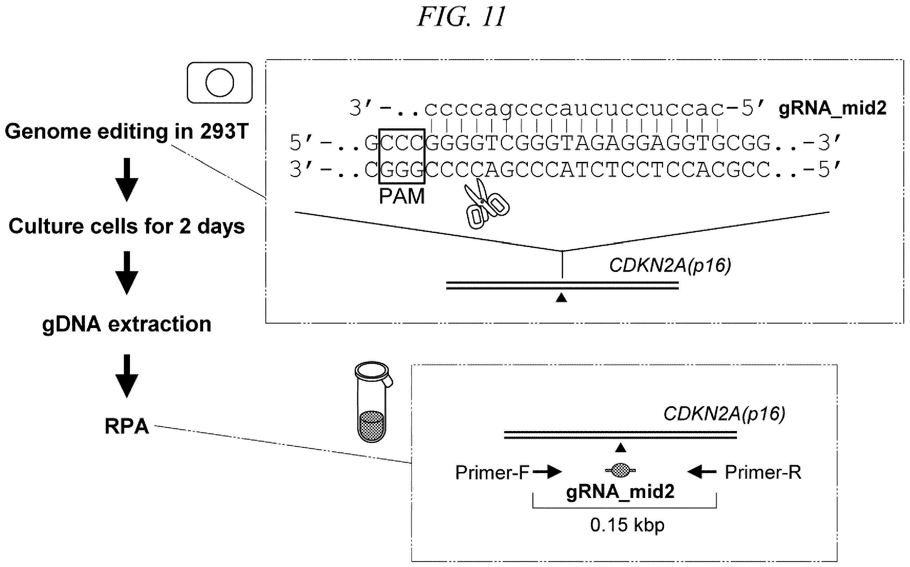

Genome editing of the *CDKN2A (p16)* gene of 293T cells was conducted. The edited genome was subjected to an RPA reaction in the presence of dCas9 and a gRNA (gRNA_mid2, SEQ ID NOS: 5 and 6) targeting the wild-type nucleic acid, and only the genome-edited nucleic acid was amplified and detected. FIG. 11 schematically illustrates the genome editing of the *CDKN2A (p16)* gene.

In the case where genome editing has not occurred, the wild-type nucleotide sequence is retained, and therefore RPA amplification is inhibited by the gRNA_mid2 and dCas9. In contrast, when genome editing has occurred and the nucleotide sequence has changed, the amplification inhibitory effect of the gRNA_mid2 and dCas9 does not occur, and RPA amplification is observed.

First, the genome editing was conducted in the following manner. First, 2 µg of a Cas9 expression plasmid (#41815, available from Addgene) and 2 µg of an sgRNA expression plasmid (sgRNA_mid2) (Non-Patent Document 5) targeting the human *CDKN2A (p16)* gene were transfected into 293T cells (4×10⁵ cells) using a transfection reagent "Lipofectamine 3000" (manufactured by Thermo Fisher Scientific Inc.). After culturing for two days, the cells were collected, and the genome DNA was extracted and purified.

Subsequently, with the exceptions of using a total mass of 100 ng of the obtained genome DNA as a template, and using a forward primer (SEQ ID NO: 9) and a reverse primer (SEQ ID NO: 10) for conducting RPA amplification of the region, within the genome DNA of the *CDKN2A (p16)* gene of the 293T cells, containing the nucleotide sequence targeted by the gRNA_mid2, RPA reactions were conducted in the same manner as Example 1, and following purification, electrophoresis was conducted. The proportion of genome-edited genome DNA was reduced by mixing a small amount of the 293T gDNA that had undergone genome editing with 293T gDNA that had not been subjected to genome editing.

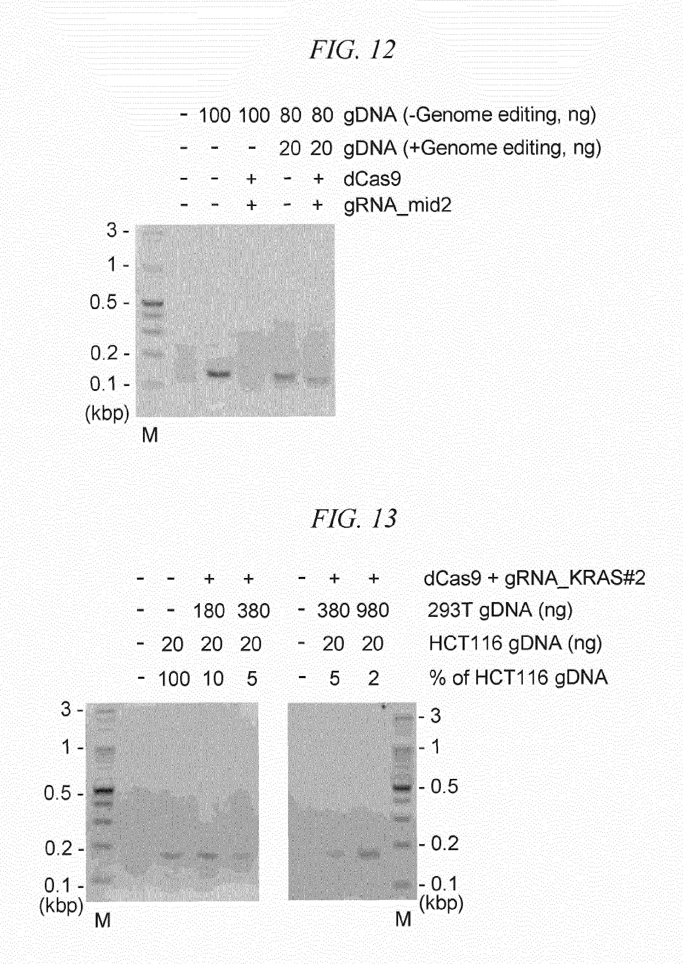

The electrophoresis results are shown in FIG. 12. In the reaction mixture containing only the 293T gDNA that had not been subjected to genome editing as the template, no amplification product was confirmed, indicating that the nucleic acid amplification reaction had been inhibited by the gRNA_mid2. In contrast, in the reaction mixture containing a mixture of the 293T gDNA that had not been subjected to genome editing (80 ng) and the 293T gDNA that had undergone genome editing (20 ng) as the template, an amplification product was confirmed. Confirmation of the nucleotide sequences of the amplification products obtained in the various reaction mixtures revealed that the amplification product in the reaction mixture that used the 293T gDNA that had not been subjected to genome editing as a template had the same sequence as the wild-type nucleic acid sequence of the *CDKN2A (p16)* gene. The amplification product in the reaction mixture that used a mixture of the 293T gDNA that had not been subjected to genome editing and the 293T gDNA that had undergone genome editing as a template was predominantly of the wild-type nucleic acid sequence, but also contained a small amount of highly diverse nucleotide sequences thought to be genome-edited sequences. On the other hand, in the amplification product from the reaction mixture that used a mixture of the 293T gDNA that had not been subjected to genome editing and the 293T gDNA that had undergone genome editing as a template, and also contained added dCas9 and gRNA_mid2, only the highly diverse nucleotide sequences were detected, and the wild-type nucleic acid sequence was not amplified.

### [Example 5]

By mixing a small amount of HCT116 gDNA with 293T gDNA, and using the resulting nucleic acid with a reduced abundance ratio of mutant *KRAS (G13D)* ([amount of gDNA derived from cells having mutant *KRAS (G13D)*] / ([amount of gDNA derived from cells having mutant *KRAS (G13D)*] + [amount of gDNA derived from cells having only wild-type *KRAS*]) × 100%) as a template, an investigation was conducted as to whether or not RPA amplification of wild-type *KRAS* could be inhibited by gRNA_KRAS#2 and dCas9.

With the exceptions of using, as the template, mixtures of 293T gDNA and HCT116 gDNA in which the amount of HCT116 gDNA was kept constant at 20 ng but the abundance ratio of mutant *KRAS (G13D)* was varied as shown in FIG. 13, and using gRNA_KRAS#2 as the gRNA, RPA reactions were conducted in the same manner as Example 1, and following purification, electrophoresis was conducted.

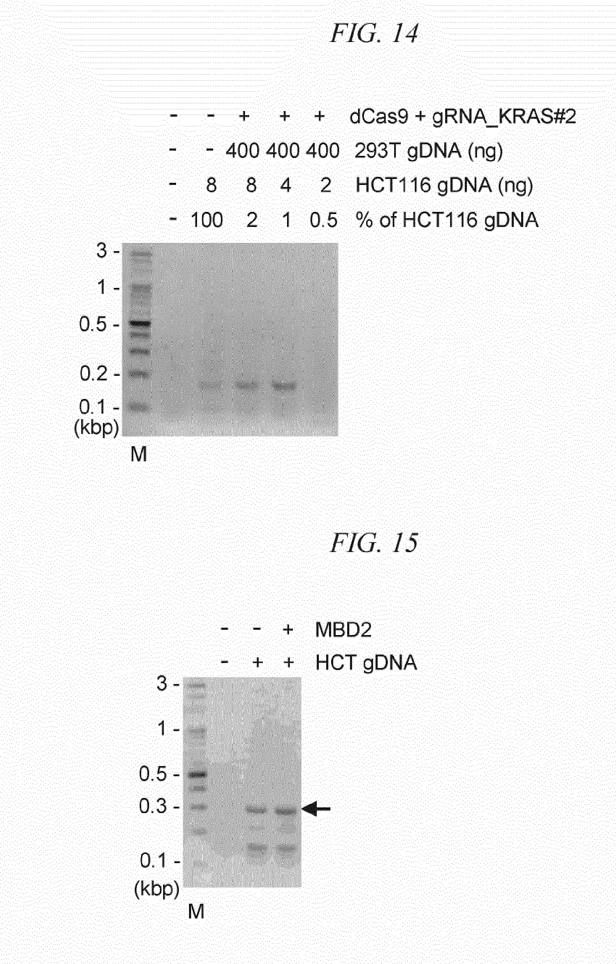

The electrophoresis results are shown in FIG. 14. Even in the reaction mixture where the abundance ratio of the gDNA derived from cells having mutant *KRAS (G13D)* was only 2%, a nucleic acid amplification product was confirmed. Confirmation of the nucleotide sequences of the various amplification products revealed that in the amplification products of the reaction mixtures in which the abundance ratio of gDNA derived from cells having mutant *KRAS (G13D)* was within a range from 5 to 100%, only mutant *KRAS (G13D)* was detected, whereas in the amplification product of the reaction mixture in which the abundance ratio of gDNA derived from cells having mutant *KRAS (G13D)* was 2%, both the mutant *KRAS (G13D)* and the wild-type *KRAS* were confirmed.

Subsequently, with the exceptions of using, as the template, mixtures of 293T gDNA and HCT116 gDNA in which the amount of 293T gDNA was kept constant at 400 ng but the abundance ratio of mutant *KRAS (G13D)* was varied as shown in FIG. 14, and using gRNA_KRAS#2 as the gRNA, RPA reactions were conducted in the same manner as Example 1, and following purification, electrophoresis was conducted.

The electrophoresis results are shown in FIG. 14. Even in the reaction mixture where the abundance ratio of the gDNA derived from cells having mutant *KRAS (G13D)* was only 1 %, a nucleic acid amplification product was confirmed. Confirmation of the nucleotide sequences of the various amplification products revealed that in the amplification products of the reaction mixtures in which the abundance ratio of gDNA derived from cells having mutant *KRAS (G13D)* was within a range from 1 to 100%, only mutant *KRAS (G13D)* was detected. These results indicated that even when the abundance ratio of the target nucleic acid (in these tests, the mutant *KRAS (G13D)*) was as low as 1 %, the target nucleic acid could still be detected.

### [Example 6]

In the *CDKN2A (p14ARF)* gene of HCT116 cells, one G is deleted in only one allele (Gx4). Further, in the *CDKN2A (p14ARF)* gene of HCT116 cells, although the cytosine in the vicinity of the Gx4 location in the Gx4 allele has not undergone methylation modification, the cytosine in the vicinity of the Gx5 location in the Gx5 allele has undergone methylation modification. Accordingly, by adding a CpG methylated DNA-binding protein to the RPA reaction mixture, RPA amplification using the Gx5 allele as a template was inhibited, and the Gx4 allele was detected. An MBD2 protein ("EpiXplore (a registered trademark) Methylated DNA Enrichment Kit" manufactured by Takara Bio Inc.) was used as the CpG methylated DNA-binding protein.

With the exception of using 0.25 µg of the MBD2 protein instead of the gRNA and dCas9, RPA reactions were conducted in the same manner as Example 1, and following purification, electrophoresis was conducted. A forward primer (SEQ ID NO: 11) and a reverse primer (SEQ ID NO: 12) for amplifying the Gx4·Gx5 location of the *CDKN2A (p14ARF)* gene were used as a primer set. Neither primer included CpG.

The electrophoresis results are shown in FIG. 15. In the reaction mixture containing no added MBD2, a nucleic acid amplification product was confirmed. An amplification product was also observed when MBD2 was added. Confirmation of the nucleotide sequences of the various amplification products revealed that the amplification product in the reaction mixture containing no added MBD2 contained both Gx4-type p14ARF and Gx5-type p14ARF. In contrast, the amplification product of the reaction mixture to which MBD2 had been added contained only the Gx4-type p14ARF, and nucleic acid amplification of the Gx5-type p14ARF could not be confirmed. These results indicated that by including a CpG methylated DNA-binding protein in the RPA reaction mixture, nucleic acid amplification with a CpG methylated nucleic acid as the template could be inhibited, and a target nucleic acid that is not CpG methylated could be detected.

Within the *CDKN2A (p16)* gene of HCT116 cells, although the cytosine in the vicinity of the Gx5 location in the Gx5 allele has not undergone methylation modification, the cytosine in the vicinity of the Gx4 location in the Gx4 allele has undergone methylation modification. Accordingly, by adding a CpG methylated DNA-binding protein to the RPA reaction mixture, RPA amplification using the Gx4 allele as a template was inhibited, and the Gx5 allele was detected. An MBD2 protein was used as the CpG methylated DNA-binding protein.

With the exception of using 0.5 µg of the MBD2 protein instead of the gRNA and Cas9, RPA reactions were conducted in the same manner as Example 3, and following purification, electrophoresis was conducted. A forward primer (SEQ ID NO: 13) and a reverse primer (SEQ ID NO: 14) for amplifying the Gx4·Gx5 location of the *CDKN2A (p16)* gene were used as a primer set. The reverse primer (SEQ ID NO: 14) included one CpG.

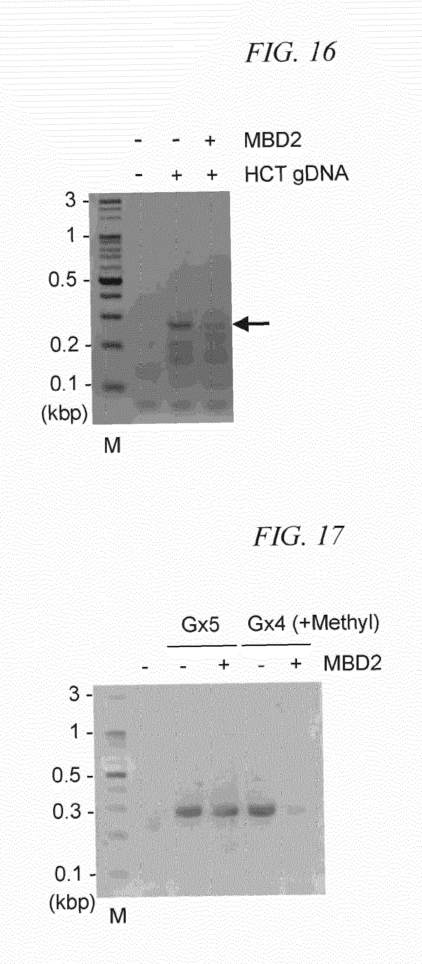

The electrophoresis results are shown in FIG. 16. In the reaction mixture containing no added MBD2, a nucleic acid amplification product was confirmed. An amplification product was also observed when MBD2 was added. Confirmation of the nucleotide sequences of the various amplification products revealed that the amplification product in the reaction mixture containing no added MBD2 contained both Gx5-type p16 and Gx4-type p16. In contrast, the amplification product of the reaction mixture to which MBD2 had been added contained only the Gx5-type p16, and nucleic acid amplification of the Gx4-type p16 could not be confirmed. These results also indicated that by including a CpG methylated DNA-binding protein in the RPA reaction mixture, nucleic acid amplification with a CpG methylated nucleic acid as the template could be inhibited, and a target nucleic acid that is not CpG methylated could be detected.

Next, an investigation was conducted as to whether or not nucleic acid amplification was inhibited by this method even for DNA that had undergone artificial CpG methylation in a test tube. First, using a forward primer (SEQ ID NO: 15) and a reverse primer (SEQ ID NO: 16) that sandwiched the Gx4·Gx5 location of the *CDKN2A (p14ARF)* gene, the nucleotide sequence sandwiched by the two primers was amplified by PCR. For the PCR, "AmpliTaq Gold (a registered trademark) 360 Master Mix" (manufactured by Thermo Fisher Scientific Inc.) was used, and a PCR reaction mixture was prepared containing 10 ng of HCT116 genome DNA in 10 µL and 0.5 µM of each primer. The reaction was conducted by first performing denaturation at 95°C for 10 minutes, subsequently conducting 30 cycles of 15 seconds at 95°C, 30 seconds at 60°C and 30 seconds at 72°C, and then conducting a treatment at 72°C for one minute. The amplification product was purified using a "PCR/Gel DNA purification kit" (manufactured by Nippon Genetics Co., Ltd.), subsequently cloned to T-Vector pMD20 (manufactured by Takara Bio Inc.), and then amplified with Competent Quick DH5a (manufactured by Toyobo Co., Ltd.). The amplified plasmid was purified using "NucleoBond (a registered trademark) Xtra Midi Plus" (manufactured by Takara Bio Inc.). Two types of the purified plasmid were prepared, one containing Gx4 (p14_Gx4 plasmid) and one containing Gx5 (p14_Gx5 plasmid).

Within the HCT116 cells, although the cytosine in the vicinity of the Gx4 location in the *CDKN2A (p14ARF)* gene has not undergone methylation modification, the cytosine in the vicinity of the Gx5 location has undergone methylation modification. However, in the plasmid purified from E. coli, these modifications have not occurred. Accordingly, the cytosine in the vicinity of the Gx4 location rather than the vicinity of the Gx5 location was intentionally subjected to methylation modification in a test tube. Specifically, 1 µg of the purified p14_Gx4 plasmid was subjected to a methylation treatment by reaction at 37°C for one hour with 6 units of a CpG methyltransferase M.SssI (manufactured by New England Biolabs Ltd.) and 160 µM of S-adenosyl methionine. The methylated p14_Gx4 plasmid and the unmethylated p14_Gx5 plasmid were purified using a "PCR/Gel DNA purification kit" (manufactured by Nippon Genetics Co., Ltd.).

Using 1 pg of plasmid DNA instead of the genome DNA, an RPA reaction preparatory solution was prepared in the same manner as Example 1. Following addition of 0.5 µg of the MBD2 protein to the RPA reaction preparatory solution, the resulting mixture was incubated at 37°C for 10 minutes, 1 µL of a 280 mM MgOAc solution was then added, and an RPA reaction was conducted by incubation at 37°C for 10 minutes. A forward primer (SEQ ID NO: 11) and a reverse primer (SEQ ID NO: 12) for amplifying the Gx4·Gx5 location of the *CDKN2A (p14ARF)* gene were used as a primer set. Following reaction, 2 µL of the reaction mixture was subjected to electrophoresis without purification.

The electrophoresis results are shown in FIG. 17. In the reaction mixture containing no added MBD2, a nucleic acid amplification product was confirmed. When MBD2 was added, nucleic acid amplification was observed from the unmethylated p14_Gx5 plasmid, but nucleic acid amplification from the methylated p14_Gx4 plasmid was inhibited. These results also indicated that an artificially CpG methylated DNA could also be used as a template, and that CpG methylation was directly involved in the inhibition of nucleic acid amplification.

### [Example 7]

An investigation was conducted as to whether or not the RPA reaction was inhibited by other DNA-binding proteins besides dCas9/gRNA and MBD2. The chicken DT40#205-2 cell line has, in the *Pax5* gene promoter region, LexA-binding sequences which bind to the bacterial DNA-binding protein LexA protein (Non-Patent Document 6). Accordingly, using DT40#205-2 cell genome DNA as a template, an investigation was conducted as to whether or not RPA amplification of the LexA-binding sequences were inhibited when the LexA protein was added to the RPA reaction mixture.

With the exception of using 20 ng of LexA protein instead of the gRNA and dCas9, an RPA reaction was conducted in the same manner as Example 1, and following purification, electrophoresis was conducted. A LexA protein DNA-binding domain synthesized by Sysmex Corporation (ProCube, production number: 13T_0170) was used as the LexA protein. Further, 20 ng of dCas9 protein was used as a negative control protein. A forward primer (SEQ ID NO: 17) and a reverse primer (SEQ ID NO: 18) for amplifying the region containing the LexA-binding sequences were used as a primer set.

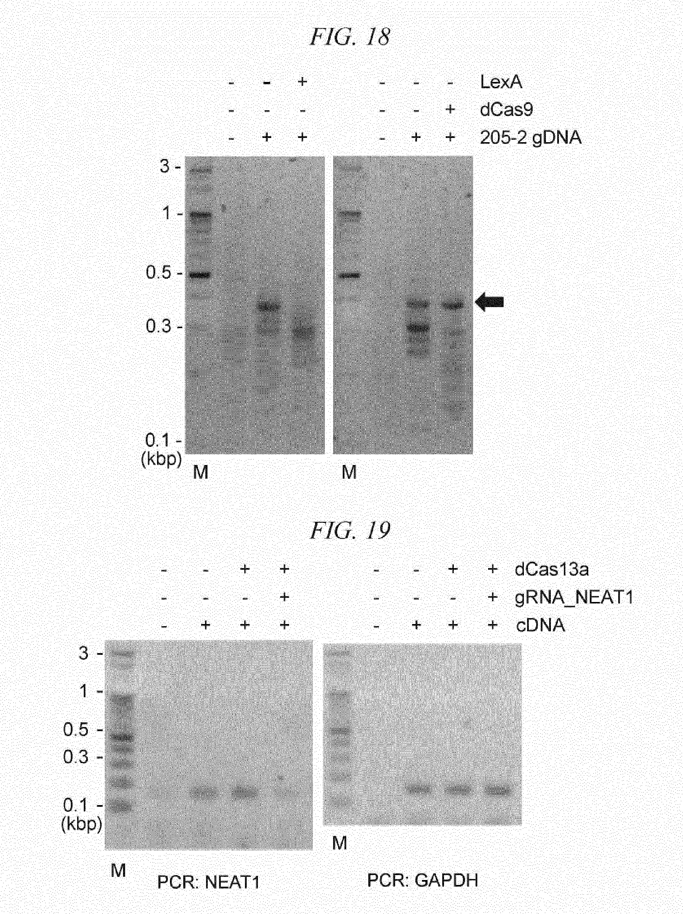

The electrophoresis results are shown in FIG. 18. In the reaction mixture containing no added LexA protein, nucleic acid amplification was observed. When the LexA protein was added, nucleic acid amplification from the genome DNA was inhibited. In the case of the dCas9 addition as a negative control, nucleic acid amplification from the genome DNA was not inhibited. These results indicated that DNA-binding proteins other than CRISPR complexes and MBD2 protein were also able to inhibit amplification of a target nucleic acid. By using this technique, an evaluation can be made as whether or not a specific DNA-binding molecule has binding ability to a specific nucleotide sequence. Further, the technique can also be used for detecting whether or not a molecule having binding ability to a specific nucleotide sequence exists within a group of molecules.

### [Example 8]

Using RT-PCR, a single-stranded RNA in a nucleic acid test sample was detected as a target nucleic acid. The mRNA of the human *NEAT1* gene (NEAT1-RNA) was used as the target nucleic acid.

### [gRNA and Primers]

In this test, single-stranded RNAs composed of the nucleotide sequences shown in Table 3 were used as the gRNA. Within the gRNA nucleotide sequences shown in Table 3, the region depicted in uppercase letters is the region (RNA-binding region) that is complementary to a partial region of the target nucleic acid NEAT1-RNA. The gRNA used was chemically synthesized by Gene Design Inc. The gRNA used in the test was prepared in advance by mixing 1µL of 10 µM gRNA and 3 µL of nuclease-free water, incubating the mixture at 100°C for two minutes, and then cooling the mixture to room temperature.

**[Table 3]**

| RNA | Sequence (5' → 3') | SEQ ID NO: |
|---|---|---|
| gRNA_NEAT1 | gauuuagacuaccccaaaaacgaaggggacuaaaacCAUC AAUCUGCGUUGUGGCAUCAACGUU | 19 |
| gRNA_NEAT1_2 | gauuuagacuaccccaaaaacgaaggggacuaaaacUAUC UCUAACCAACCCUCUCCCCUUCUUC | 20 |

In this test, primers composed of the nucleotide sequences shown in Table 4 were used. The forward primer (Human NEAT1-F2) and the reverse primer (Human NEAT1 - R2) are primers designed to sandwich a cDNA sequence complementary to the RNA sequence targeted by dCas13a/gRNA_NEAT1. The forward primer (MY-0119) and the reverse primer (MY-0129) are primers designed to sandwich a cDNA sequence complementary to the RNA sequence targeted by dCas13a/gRNA_NEAT1_2. As a control, the forward primer (hGAPDH-dCas13a-F3) and the reverse primer (hGAPDH-dCas13a-R3) were used for amplifying a template composed of cDNA of the mRNA of the human *GAPDH* gene. The primers used were chemically synthesized by Eurofins Scientific S.E.

**[Table 4]**

| Primer | Sequence (5' → 3') | SEQ ID NO: |
|---|---|---|
| Human NEAT1-F2 | ctaaattgagcctccggtca | 21 |
| Human NEAT1-R2 | acaagaaggcaggcaaacag | 22 |
| MY-0119 | cactggtactgggagggatg | 23 |
| MY-0129 | cccttcaacctgcatttcctac | 24 |
| hGAPDH-dCas13a-F3 | gagccaaaagggtcatcatctct | 25 |
| hGAPDH-dCas13a-R3 | cacgataccaaagttgtcatgga | 26 |

### [Preparation of Nucleic Acid Test Sample]

Total RNA extracted from human-derived MRC-5 cells was used as the nucleic acid test sample. The MRC-5 cells were cultured in an E-MEM (manufactured by FUJIFILM Wako Pure Chemical Corporation) medium containing 10% FBS (fetal bovine serum) and penicillin-streptomycin (manufactured by Sigma-Aldrich Corporation). The RNA of the MRC-5 cells was extracted and purified using a commercially available RNA extraction reagent "Isogen II" (manufactured by Nippon Gene Co., Ltd.).

### [dCas13a]

The dCas13a protein used was a mutant protein synthesized by Sysmex Corporation (ProCube, production number: 17T_042), having point mutations introduced at R474A and R1046A into wild-type Cas13a derived from Leptotrichia wadei.

### [RT-PCR using gRNA_NEAT1]

First, 0.46 µL of gRNA and 0.24 µg of dCasl3a protein were mixed with nuclease-free water to prepare a sample of 5 µL, and this sample was used as a dCas13a/gRNA solution.

A reverse transcription (RT) was conducted first, using "ReverTra Ace qPCR RT Master Mix with gDNA Remover" (manufactured by Toyobo Co., Ltd.). First, 2 µL of "4×DN Master Mix" (containing added gDNA Remover), 1 ng of RNA, 5 µL of the dCas13a/gRNA solution and nuclease-free water were mixed to form a sample of 8 µL, and this sample was incubated at 37°C for 5 minutes. Subsequently, 2 µL of "5×RT Master Mix 11" was added to this reaction mixture, and the resulting mixture was incubated at 37°C for 30 minutes, subsequently at 50°C for 5 minutes, and then at 98°C for a further 5 minutes to complete the reverse transcription reaction. Following the reverse transcription reaction, 10 µL of nuclease-free water was mixed with the reaction solution to complete preparation of 20 µL of a cDNA solution.

Next, a PCR was conducted using "EmeraldAmp (a registered trademark) MAX PCR Master Mix" (manufactured by Takara Bio Inc.). A PCR reaction mixture was prepared containing 1 µL of the cDNA and 0.5 µM of the Human NEAT1-F2 primer and the Human NEAT1-R2 primer in a total volume of 10 µL. The reaction was conducted by first performing denaturation at 94°C for one minute, subsequently conducting 35 cycles of 15 seconds at 94°C, 15 seconds at 60°C and one minute at 72°C, and then conducting a treatment at 72°C for one minute.

### [RT-PCR using gRNA_NEAT1_2]

Preparation of the dCas13a/gRNA solution and RT were conducted in the same manner as the RT-PCR using gRNA_NEAT1.

Next, a PCR was conducted using "AmpliTaq Gold (a registered trademark) 360 Master Mix" (manufactured by Thermo Fisher Scientific Inc.). A PCR reaction solution was prepared containing 1 µL of the cDNA and 0.5 µM of the MY-0119 primer and MY-0129 primer in a total volume of 10 µL. The reaction was conducted by first performing denaturation at 95°C for 10 minutes, subsequently conducting 38 cycles of 15 seconds at 95°C, 30 seconds at 55°C and 30 seconds at 72°C, and then conducting a treatment at 72°C for one minute.

### [RT-PCR of GAPDH]

Amplification of *GAPDH* was conducted using "EmeraldAmp (a registered trademark) MAX PCR Master Mix" (manufactured by Takara Bio Inc.). A PCR reaction mixture was prepared containing 1 µL of the cDNA and 0.5 µM of the hGAPDH-dCas13a-F3 primer and the hGAPDH-dCas13a-R3 primer in a total volume of 10 µL. The reaction was conducted by first performing denaturation at 94°C for one minute, subsequently conducting 28 or 32 cycles of 15 seconds at 94°C, 15 seconds at 60°C and one minute at 72°C, and then conducting a treatment at 72°C for one minute.

### [Electrophoresis]

Electrophoresis of the amplification products obtained in the various amplification reactions was conducted using an agarose gel containing "SYBR (a registered trademark) Safe DNA Gel Stain" (manufactured by Thermo Fisher Scientific Inc.).

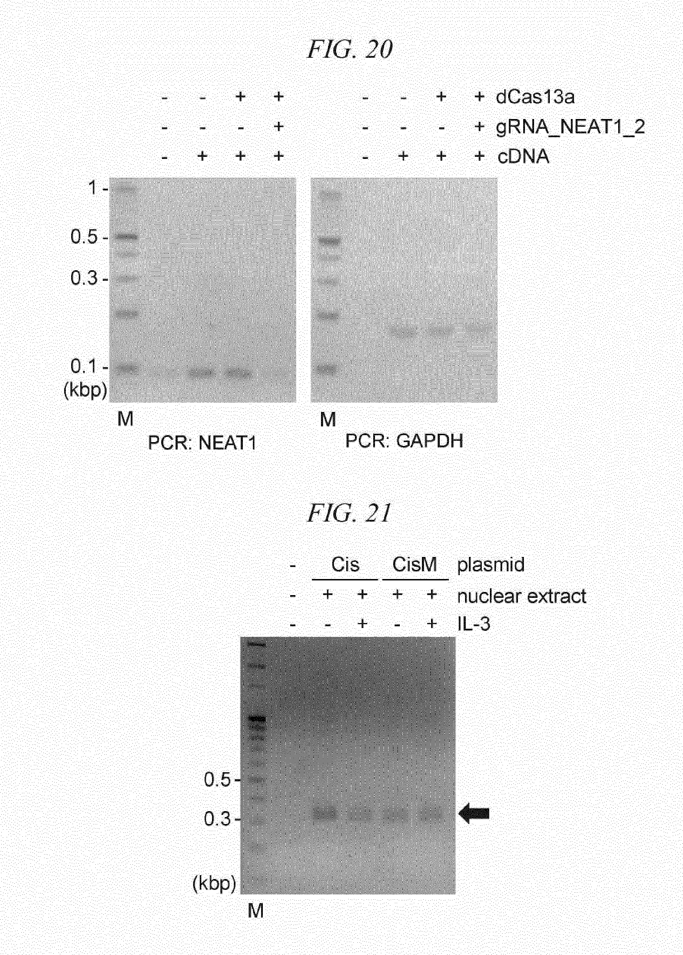

The results for the RT-PCR amplification product using gRNA_NEAT1 are shown in FIG. 19, and the results for the RT-PCR amplification product using gRNA_NEAT1_2 are shown in FIG. 20. As shown in FIG. 19, compared with the reaction mixture containing no added dCas13a and the reaction mixture to which only dCas13a had been added, the reaction mixture to which the dCas13a/gRNA_NEAT1 complex solution had been added exhibited reduced amplification of NEAT1 by PCR. On the other hand, in the case of amplification of *GAPDH,* which is not targeted by the dCas13a/gRNA_NEAT1 complex, this type of reduction in DNA amplification was not observed. Similar observations were confirmed in FIG. 20. These results indicated that the dCas13a/gRNA_NEAT1 complex specifically inhibited the reverse transcription reaction of *NEAT1*, and confirmed that by including a dCas13a/gRNA complex in the reaction mixture, a sequence-specific reverse transcription reaction could be inhibited.

### [Example 9]

The fact that Stat5 binds to a Cis promoter in Ba/F3 cells within 30 minutes of IL-3 stimulation (10 ng/mL) has already been reported (Non-Patent Document 9). Accordingly, using double-stranded DNA having Stat5-binding sites (TTCNNNGAA), and a primer set for conducting RPA amplification of the region including the Stat5-binding sites of the double-stranded DNA, Stat5 was detected in a nuclear extract of Ba/F3 cells.

### [Template Nucleic Acid]

A region (the Cis region shown in Table 5: SEQ ID NO: 27) sandwiching Stat5-binding sites in mouse Cis gene promoter was used as the template nucleic acid, and a plasmid (Cis plasmid) into which this region had been inserted was prepared. The region included four Stat5-binding sites. In SEQ ID NO: 27 shown in Table 5, the areas of white text on black background represent the Stat5-binding sites. Further, as a negative control, a mutant sequence (the CisM region shown in Table 5: SEQ ID NO: 28) in which a mutation was introduced into each Stat5-binding site to remove the Stat5-binding ability was used as the template nucleic acid, and a plasmid (CisM plasmid) into which this region had been inserted was prepared. In SEQ ID NO: 28 shown in Table 5, the areas of white text on black background represent the mutation-containing Stat5-binding sites.

**[Table 5]**

| DNA | Sequence (5' → 3') | SEQ ID NO: |
|---|---|---|
| Cis region | CAACTCTAGGAGCTCCCGCCCAGTTTTCCTGGAAA GTTCTTGGAAATCTGTCAAAGGTGTTTCCTTTCTCG GTCCAAAGCACTAGACGCCTGCACCCCCGTTCCCCT CCGGGCCGCCGCAAAGCCCGCGGTTCTAGGAAGAT GAGGCTTCCGGGAA | 27 |
| CisM region | CAACTCTAGGAGCTCCCGCCCAGTTGGCCTGGCCA GGGCTTGGCCATCTGTCAAAGGTGTTTCCTTTCTCG GTCCAAAGCACTAGACGCCTGCACCCCCGTTCCCCT CCGGGCCGCCGCAAAGCCCGCGGGCTAGGCCGAT GAGGCGGCCAA | 28 |

First, with the genome DNA of Ba/F3 cells as the template, a forward primer (mCis_-259/-199_F) and a reverse primer (mCis_-188/-104_R) sandwiching the aforementioned Cis region in the mouse Cis gene promoter were used to amplify the nucleotide sequence sandwiched between the two primers by PCR. The PCR was conducted using "EmeraldAmp (a registered trademark) MAX PCR Master Mix" (manufactured by Takara Bio Inc.), and a PCR reaction mixture was prepared containing 10 ng of Ba/F3 genome DNA and 0.5 µM of each primer in a total volume of 10 µL. The reaction was conducted by first performing denaturation at 94°C for one minute, subsequently conducting 35 cycles of 15 seconds at 94°C, 15 seconds at 60°C and one minute at 72°C, and then conducting a treatment at 72°C for one minute.

The thus obtained amplification product was purified using a "PCR/Gel DNA purification kit" (manufactured by Nippon Genetics Co., Ltd.), and was subsequently cloned to T-Vector pMD20 (manufactured by Takara Bio Inc.) and then amplified with "Competent Quick DH5a" (manufactured by Toyobo Co., Ltd.). The amplified plasmid was purified using "NucleoSpin (a registered trademark) Plasmid QuickPure" (manufactured by Takara Bio Inc.).

The CisM plasmid was produced in the following manner. Using the Cis plasmid as a template, a forward primer (mCis_-259/-199_mut_F) and a reverse primer (mCis_-188/-104_mut_R) sandwiching the Stat5-binding site were used to amplify the nucleotide sequence sandwiched between the two primers by PCR. The PCR was conducted using "EmeraldAmp (a registered trademark) MAX PCR Master Mix" (manufactured by Takara Bio Inc.), and a PCR reaction mixture was prepared containing 1 pg of the Cis plasmid and 0.5 µM of each primer in a total volume of 10 µL. The reaction was conducted by first performing denaturation at 94°C for one minute, subsequently conducting 30 cycles of 15 seconds at 94°C, 15 seconds at 55°C and one minute at 72°C, and then conducting a treatment at 72°C for one minute. In a similar manner to the Cis region amplification product, the amplification product was purified, introduced into T-Vector pMD20, amplified, and then purified to obtain the CisM plasmid. The results of DNA sequence analysis confirmed that although an unexpected mutation was observed on the reverse primer side in the CisM plasmid, the mutation illustrated in Table 5 had been introduced into the Stat5-binding sites.

**[Table 6]**

| Primer | Sequence (5' → 3') | SEQ ID NO: |
|---|---|---|
| mCis_-259/-199_F | CAACTCTAGGAGCTCCCGCC | 29 |
| mCis_-188/-104_R | TTCCCGGAAGCCTCATCTT | 30 |
| mCis_-259/-199_mut_F | caactctaggagctcccgcccagttGGcctggCC agGGcttggCCatctgtc | 31 |
| mCis_-188/-104_mut_R | GGcccggCCgcctcatcGGcctagCCccgcgg | 32 |

### [Preparation of Nuclear Extract of Ba/F3 Cells]

Nuclear extracts of Ba/F3 cells with no IL-3 stimulation and Ba/F3 cells that had undergone IL-3 stimulation treatment were prepared in the following manner.

Ba/F3 cells were cultured in an RPMI-1640 (manufactured by FUJIFILM Wako Pure Chemical Corporation) medium containing 10% FBS, 10 mM HEPES buffer (pH: 7.2) (manufactured by Nacalai Tesque Inc.), 1×non-essential amino acids, 1 mM sodium pyruvate (manufactured by Nacalai Tesque Inc.), 5 µM 2-mercaptoethanol (manufactured by Sigma-Aldrich Corporation), 1 ng/mL IL-3 (manufactured by Thermo Fisher Scientific Inc.), and penicillin-streptomycin (manufactured by Nacalai Tesque Inc.).

In the preparation of the nuclear extracts with no IL-3 stimulation and with IL-3 stimulation treatment, first, the Ba/F3 cells were washed three times with PBS, and the cells were then cultured for 6 hours in the above medium excluding the IL-3 (no IL-3 stimulation). Subsequently, sufficient IL-3 was added to the medium to achieve a concentration of 10 ng/mL, and the cells were cultured at 37°C for 30 minutes (IL-3 stimulation treatment). The Ba/F3 cells with no IL-3 stimulation and the Ba/F3 cells following IL-3 stimulation treatment were collected, and nuclear extracts were prepared using "NE-PCR (a registered trademark) Nuclear and Cytoplasmic Extraction Reagents" (manufactured by Thermo Fisher Scientific Inc.).

### [RPA Reaction]

RPA reactions were conducted in the following manner. First, 29.5 µL of rehydration buffer, 2.5 µL of a 10 µM forward primer (M13 Primer RV), 2.5 µL of a 10 µM reverse primer (M13 Primer M4) and nuclease-free water were added to and mixed with one tube (containing lyophilized reagent) of an RPA reagent (product name: TwistAmp (a registered trademark) Basic kit, manufactured by TwistDx Ltd.) to make a total volume of 50 µL. Subsequently, 10 µL of the prepared solution was placed in a separate tube, and following the addition of 1 pg of plasmid DNA (either the Cis plasmid or the CisM plasmid) and 3 ng of the nuclear extract, 10 mM of Tris (pH: 8.0) was added, thus preparing 11.3 µL of a reaction mixture. The reaction mixture was incubated at 37°C for 5 minutes, 1 µL of a 280 mM MgOAc solution was then added, and an RPA reaction was conducted by incubating the resulting mixture at 37°C for 30 minutes. Following the reaction, 2 µL of the reaction mixture was subjected to electrophoresis without purification.

**[Table 7]**

| Primer | Sequence (5' → 3') | SEQ ID NO: |
|---|---|---|
| M13 Primer RV | CAGGAAACAGCTATGAC | 33 |
| M13 Primer M4 | GTTTTCCCAGTCACGAC | 34 |

The electrophoresis results are shown in FIG. 21. In the RPA reaction using the Cis plasmid as a template, the reaction mixture containing the added nuclear extract that had undergone IL-3 stimulation treatment exhibited reduced DNA amplification by RPA compared with the reaction mixture containing the added nuclear extract with no IL-3 stimulation. This is because the Stat5 that existed in the nuclear extract following IL-3 stimulation treatment bound to the Stat5-binding sites in the Cis plasmid, thereby inhibiting DNA amplification, whereas no Stat5 existed in the nuclear extract with no IL-3 stimulation, meaning the RPA reaction was not inhibited. On the other hand, in the RPA reaction using the CisM plasmid as a template, a similar level of DNA amplification was observed regardless of whether or not IL-3 stimulation was conducted. The above results indicated that nucleic acid amplification by RPA could be used to evaluate whether or not a protein that binds to a target DNA existed in a solution such as a cell lysate.

### [Example 10]

Using a genome DNA library as a template, an RPA reaction was conducted in the presence of a CpG methylated DNA-binding protein, and an investigation was conducted as to whether or not DNA that was not CpG methylated was amplified.

### [Genome DNA Library]

A DNA library was produced by Promega Corporation using genome DNA extracted from HCT116 cells and purified (product number: KK0500). Index15 (ATGTCA) for next-generation sequencing by Illumina, Inc. was included in the adapter sequence added during DNA library production. Addition of the adapter was performed without using a DNA amplification operation.

### [RPA Reaction]

An RPA reaction was conducted in the following manner, using primers complementary to the adapter sequence. First, 29.5 µL of rehydration buffer, 2.5 µL of a 10 µM forward primer (P5-NGS-Lib-Promega-RPA-F), and 2.5 µL of a 10 µM reverse primer (P7-NGS-Lib-Promega-RPA-R) and 9.5 µL of nuclease-free water were added to and mixed with one tube (containing lyophilized reagent) of an RPA reagent (product name: TwistAmp (a registered trademark) Basic kit, manufactured by TwistDx Ltd.). Subsequently, 17.5 µL of the prepared solution was placed in a separate tube, and 0.5 µL of the DNA library, 0.5 µg of an MBD2 protein ("EpiXplore (a registered trademark) Methylated DNA Enrichment Kit", manufactured by Takara Bio Inc.) and nuclease-free water were added, thus preparing 19 µL of an RPA reaction preparatory solution. This RPA reaction preparatory solution was incubated at 37°C for 10 minutes, 1 µL of a 280 mM MgOAc solution was then added, and an RPA reaction was conducted by incubating the mixture at 37°C for 10 minutes. Following completion of the reaction, a "PCR/Gel DNA purification kit" (manufactured by Nippon Genetics Co., Ltd.) was used to purify the nucleic acids from the reaction mixture.

### [Analysis of Amplification Product of RPA Reaction]

In the *CDKN2A (pl4ARF)* gene of HCT116 cells, one G is deleted in only one allele (Gx4). Further, in the *CDKN2A (p14ARF)* gene of HCT116 cells, although the cytosine in the vicinity of the Gx4 location in the Gx4 allele has not undergone methylation modification, the cytosine in the vicinity of the Gx5 location in the Gx5 allele has undergone methylation modification. Accordingly, whether RPA amplification with the Gx5 allele as a template was inhibited when the MBD2 protein was added to the RPA reaction mixture was confirmed by PCR. Specifically, in order to amplify the *CDKN2A (pl4ARF)* gene, a forward primer (hp14ARF-Ex1-F) and a reverse primer (hp14ARF-Ex1-R) were used to amplify the nucleotide sequence sandwiched by the two primers by PCR. The PCR was conducted using "AmpliTaq Gold (a registered trademark) 360 Master Mix" (manufactured by Thermo Fisher Scientific Inc.). A PCR reaction mixture was prepared containing 0.5 µL of the RPA reacted DNA and 0.5 µM of the hp14ARF-Ex1-F primer and the hp14ARF-Ex1-R primer in 10 µL. The PCR reaction was conducted by first performing denaturation at 95°C for 10 minutes, subsequently conducting 30 cycles of 15 seconds at 95°C, 30 seconds at 60°C and 30 seconds at 72°C, and then conducting a treatment at 72°C for one minute.

**[Table 8]**

| Primer | Sequence (5' → 3') | SEQ ID NO: |
|---|---|---|
| P5-NGS-Lib-Promega-RPA-F | ATACGGCGACCACCGAGATCtacactctttccc | 35 |
| P7-NGS-Lib-Promega-RPA-R | CAAGCAGAAGACGGCATACGAGattctgacatg | 36 |
| hp14ARF-Ex1-F | agtgagggttttcgtggttcac | 37 |
| hp14ARF-Ex1-R | cctagacgctggctcctcagta | 38 |

Following the PCR reaction, 2 µL of the reaction mixture was subjected to electrophoresis without purification. The results confirmed that a PCR amplification product was obtained regardless of whether or not the MBD2 protein was added to the RPA reaction. Confirmation of the nucleotide sequences of the various amplification products revealed that when PCR was conducted using, as a template, the DNA of the amplification product from the RPA reaction in which the MBD2 protein was not added, both the Gx4-type p14ARF and the Gx5-type p14ARF were amplified. In contrast, when the RPA reaction was conducted following addition of the MBD2 protein, and the obtained DNA of the amplification product from the RPA reaction was used as a template to conduct PCR, the Gx4-type p14ARF was amplified preferentially, whereas amplification of the Gx5-type p14ARF decreased. These results indicated that when a genome DNA library was used as a template, and a primer set capable of amplifying the entire library was used, by introducing a CpG methylated DNA-binding protein into the RPA reaction mixture nucleic acid amplification with CpG methylated DNA as a template was inhibited, and amplification of only DNA that was not CpG methylated was enabled.

Using the amplification product following an RPA reaction as a template, the existence or absence of CpG methylated DNA amplification was confirmed by PCR amplification and DNA sequence analysis (Sanger sequencing), but next-generation sequence analysis may also be used instead of DNA sequence analysis. In other words, by using next-generation sequencing to comprehensively analyze the nucleotide sequence of the amplification product of the RPA reaction conducted in the presence of the MBD2 protein using the genome DNA library as a template, and performing a comparison with the nucleotide sequence of the amplification product of the RPA reaction conducted in the absence of the MBD2 protein using the genome DNA library as a template, CpG methylated sites on the genome can be comprehensively identified.

## Claims

1. A method for detecting a target nucleic acid that discriminates the target nucleic acid from a non-target nucleic acid having a nucleotide sequence or modification state that differs from a portion of the target nucleic acid, the method comprising conducting a nucleic acid amplification reaction:
using a region in the non-target nucleic acid in which the nucleotide sequence or modification state differs from that of the target nucleic acid as a target region,
using a region in the target nucleic acid in which the nucleotide sequence or modification state differs from that of the non-target nucleic acid as a corresponding target region,
using a nucleic acid test sample as a template, and
using a primer that hybridizes with both the target nucleic acid and the non-target nucleic acid,
with the nucleic acid amplification reaction conducted in presence of a molecule (excluding molecules composed solely of a nucleic acid) capable of binding specifically to the target region in the non-target nucleic acid, under temperature conditions under which the molecule can bind to the non-target nucleic acid, and then
detecting the target nucleic acid based on the presence or absence of an amplification product.

2. The method for detecting a target nucleic acid according to Claim 1, wherein when an amplification product is obtained in the nucleic acid amplification reaction, the target nucleic acid is contained in the nucleic acid test sample.

3. The method for detecting a target nucleic acid according to Claim 1 or 2, wherein the nucleic acid amplification reaction is conducted under temperature conditions of 65°C or lower.

4. The method for detecting a target nucleic acid according to any one of Claims 1 to 3, wherein the nucleic acid amplification reaction is an isothermal nucleic acid amplification reaction.

5. The method for detecting a target nucleic acid according to any one of Claims 1 to 4, wherein the nucleic acid amplification reaction uses a Recombinase Polymerase Amplification method.

6. The method for detecting a target nucleic acid according to any one of Claims 1 to 5, wherein the corresponding target region in the target nucleic acid and the target region in the non-target nucleic acid have different nucleotide sequences.

7. The method for detecting a target nucleic acid according to Claim 6, wherein the target region is a mutation site of a gene mutation or a polymorphic site of a gene polymorphism.

8. The method for detecting a target nucleic acid according to any one of Claims 1 to 7, wherein the molecule is a complex of a DNA strand cleavage activity-deficient Cas9 protein and a gRNA.

9. The method for detecting a target nucleic acid according to Claim 8, wherein the gRNA specifically recognizes and binds to DNA having a nucleotide sequence complementary to the target region in the non-target nucleic acid.

10. The method for detecting a target nucleic acid according to any one of Claims 1 to 9, wherein
the nucleic acid test sample has been subjected to a bisulfite treatment, and
methylation states of the corresponding target region and the target region differ, and include bases that yield a difference between the corresponding target region and the target region as a result of the bisulfite treatment.

11. The method for detecting a target nucleic acid according to any one of Claims 1 to 7, wherein the molecule is a complex of an RNA strand cleavage activity-deficient Cas13a protein and a gRNA.

12. The method for detecting a target nucleic acid according to Claim 11, wherein the gRNA specifically recognizes and binds to RNA having a nucleotide sequence complementary to the target region in the non-target nucleic acid.

13. The method for detecting a target nucleic acid according to any one of Claims 1 to 5, wherein the corresponding target region in the target nucleic acid and the target region in the non-target nucleic acid have different modification states.

14. The method for detecting a target nucleic acid according to Claim 13, wherein
the corresponding target region in the target nucleic acid is a region that has not undergone CpG methylation modification,
the target region in the non-target nucleic acid is a region that has undergone CpG methylation modification, and
the molecule is a CpG methylated DNA-binding protein.

15. A target nucleic acid detection kit used in the method for detecting a target nucleic acid according to any one of Claims 8 to 10, the kit having
a primer that hybridizes with both the target nucleic acid and the non-target nucleic acid,
a DNA strand cleavage activity-deficient Cas9 protein, and
a gRNA.

16. A target nucleic acid detection kit used in the method for detecting a target nucleic acid according to Claim 14, the kit having
a primer that hybridizes with both the target nucleic acid and the non-target nucleic acid, and
a CpG methylated DNA-binding protein.

17. The target nucleic acid detection kit according to Claim 15 or 16, further including a recombinase, a single-stranded DNA-binding protein, and a DNA polymerase.

18. A target nucleic acid detection kit used in the method for detecting a target nucleic acid according to Claim 11 or 12, the kit having
a primer that hybridizes with both the target nucleic acid and the non-target nucleic acid,
an RNA strand cleavage activity-deficient Cas13a protein, and
a gRNA.

19. A method for detecting a nucleic acid-binding molecule, the method comprising:
conducting a nucleic acid amplification reaction using a test sample, a nucleic acid, and a primer that hybridizes with the nucleic acid, wherein
when the nucleic acid-binding molecule is contained within the test sample, an amplification reaction product is not obtained from the nucleic acid amplification reaction, whereas when the nucleic acid-binding molecule is not contained within the test sample, an amplification reaction product is obtained from the nucleic acid amplification reaction.

20. The method for detecting a nucleic acid-binding molecule according to Claim 19, wherein the nucleic acid-binding molecule is a molecule that binds to a specific nucleic acid sequence.

21. The method for detecting a nucleic acid-binding molecule according to Claim 19, wherein the nucleic acid-binding molecule is a CpG methylated DNA-binding protein.

22. A nucleic acid-binding molecule detection kit used in the method for detecting a nucleic acid-binding molecule according to any one of Claims 19 to 21, the kit having
a nucleic acid, and
a primer that hybridizes with the nucleic acid.

23. A method for evaluating nucleic acid-binding ability to a nucleic acid of a test substance, the method comprising
conducting a nucleic acid amplification reaction, using a test substance, a target nucleic acid that evaluates nucleic acid-binding ability of the test substance, and a primer that hybridizes with the nucleic acid, under temperature conditions under which the test substance can bind to the nucleic acid, wherein
when an amplification product is obtained, the test substance is evaluated as not having binding ability to the nucleic acid, whereas when an amplification product is not obtained, the test substance is evaluated as having binding ability to the nucleic acid.

24. The method for evaluating nucleic acid-binding ability according to Claim 23, wherein the test substance is a complex of a DNA strand cleavage activity-deficient Cas9 protein and a gRNA.

25. The method for evaluating nucleic acid-binding ability according to Claim 23, wherein the nucleic acid is a CpG methylated DNA.

26. A nucleic acid-binding ability evaluation kit used in the method for evaluating nucleic acid-binding ability according to any one of Claims 23 to 25, the kit having
a target nucleic acid that evaluates nucleic acid-binding ability of the test substance, and
a primer that hybridizes with the nucleic acid.
